(19) Europäisches Patentamt
European Patent Office
Office européen des brevets

(11) EP 2 945 608 B1

(12) **EUROPEAN PATENT SPECIFICATION**

(45) Date of publication and mention of the grant of the patent:
**23.05.2018 Bulletin 2018/21**

(21) Application number: **14701336.1**

(22) Date of filing: **20.01.2014**

(51) Int Cl.:
*A61Q 17/04* (2006.01) *A61Q 19/08* (2006.01)
*A61Q 19/02* (2006.01) *A61K 8/41* (2006.01)
*A61K 8/86* (2006.01) *A61K 8/06* (2006.01)

(86) International application number:
**PCT/EP2014/051012**

(87) International publication number:
**WO 2014/111565 (24.07.2014 Gazette 2014/30)**

(54) **COSMETIC OR DERMATOLOGICAL EMULSION COMPRISING A MEROCYANINE AND AN EMULSIFYING SYSTEM CONTAINING A GEMINI SURFACTANT**

KOSMETISCHE ODER DERMATOLOGISCHE EMULSION MIT EINEM MEROCYANIN UND EMULGIERUNGSSYSTEM MIT EINEM GEMINI-TENSID

COMPOSITION COSMÉTIQUE OU DERMATOLOGIQUE COMPRENANT UNE MÉROCYANINE ET UN SYSTÈME ÉMULSIONNANT CONTENANT UN TENSIOACTIF GÉMINÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.01.2013 FR 1350484**

(43) Date of publication of application:
**25.11.2015 Bulletin 2015/48**

(73) Proprietor: **L'Oréal**
**75008 Paris (FR)**

(72) Inventors:
- **ROUDOT, Angelina**
**F-94270 Le Kremlin Bicêtre (FR)**
- **CANDAU, Didier**
**F-91570 Bievres (FR)**

(74) Representative: **Le Blainvaux Bellegarde, Françoise**
**L'Oréal**
**Service DIPI**
**9 Rue Pierre Dreyfus**
**92110 Clichy (FR)**

(56) References cited:
**WO-A1-2013/010590 WO-A2-2008/090066**
**WO-A2-2013/011094**

- **"Use of merocyanine derivatives as UV absorbers in cosmetic formulations (I)", IP.COM JOURNAL, IP.COM INC., WEST HENRIETTA, NY, US, 23 February 2009 (2009-02-23), XP013129682, ISSN: 1533-0001**
- **"Process for producing 3-amino-2-cyclohexan-1-ylidene compounds", IP.COM JOURNAL, IP.COM INC., WEST HENRIETTA, NY, US, 29 April 2009 (2009-04-29), XP013131313, ISSN: 1533-0001**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 945 608 B1

## Description

The present invention relates to a cosmetic or dermatological composition in emulsion form, comprising, in a physiologically acceptable support:

a) at least one aqueous phase and
b) at least one oily phase, and
c) at least one merocyanine compound of formula (1) which will be defined in greater detail herein below and
d) at least one emulsifying system containing at least one gemini surfactant.

Another subject of the present invention consists of a non-therapeutic cosmetic process for caring for and/or making up a keratin material, comprising the application, to the surface of the said keratin material, of at least one composition according to the invention as defined above.

The invention also relates to a non-therapeutic cosmetic process for limiting the darkening of the skin and/or improving the colour and/or uniformity of the complexion, comprising the application, to the surface of the keratin material, of at least one composition as defined previously.

The invention also relates to a non-therapeutic cosmetic process for preventing and/or treating the signs of ageing of a keratin material, comprising the application, to the surface of the keratin material, of at least one composition as defined previously.

It is known that radiation with wavelengths of between 280 nm and 400 nm permits tanning of the human epidermis and that radiation with wavelengths of between 280 and 320 nm, known as UV-B rays, harms the development of a natural tan. Exposure is also liable to bring about a detrimental change in the biomechanical properties of the epidermis, which is reflected by the appearance of wrinkles, leading to premature ageing of the skin.

It is also known that UV-A rays with wavelengths of between 320 and 400 nm penetrate more deeply into the skin than UV-B rays. UV-A rays cause immediate and persistent browning of the skin. Daily exposure to UVA rays, even of short duration, under normal conditions can result in damage to the collagen fibres and the elastin, which is reflected by a modification in the microrelief of the skin, the appearance of wrinkles and uneven pigmentation (liver spots, lack of uniformity of the complexion).

Protection against UVA and UVB rays is thus necessary. An efficient photoprotective product should protect against both UVA and UVB rays.

Many photoprotective compositions have been proposed to date to overcome the effects induced by UVA and/or UVB rays. They generally contain organic or mineral UV-screening agents, which function according to their own chemical nature and according to their own properties by absorption, reflection or scattering of the UV rays. They generally comprise mixtures of liposoluble organic screening agents and/or water-soluble UV screening agents in combination with metal oxide pigments, such as titanium dioxide or zinc oxide.

Many cosmetic compositions for limiting the darkening of the skin and improving the colour and uniformity of the complexion have been proposed to date. It is well known in the field of antisun products that such compositions may be obtained by using UV-screening agents, and in particular UVB-screening agents. Certain compositions may also contain UVA-screening agents. This screening system should cover UVB protection for the purpose of limiting and controlling the neosynthesis of melanin, which promotes the overall pigmentation, but should also cover UVA protection so as to limit and control the oxidation of the already-existing melanin leading to darkening of the skin colour.

However, no composition contains a particular combination of UV-screening agents that would be especially suited to photoprotecting the skin and particularly to improving the quality of the skin as regards both the colour and its mechanical elasticity properties.

Advantageously, this improvement is particularly visible on already-pigmented skin so as not to increase the melanin pigmentary load or the structure of the melanin already present in the skin.

In point of fact, the majority of the organic UV-screening agents consist of aromatic compounds which absorb in the wavelength range between 280 and 370 nm. In addition to their power for screening out sunlight, the desired photoprotective compounds should also have good cosmetic properties, good solubility in the usual solvents and in particular in fatty substances such as oils, and also good chemical stability and good photostability alone or in combination with other UV-screening agents. They should also be colourless or at least have a colour that is cosmetically acceptable to the consumer.

One of the main drawbacks known to date of these compositions is that these screening systems are insufficiently effective against UV rays and in particular against long UVA rays with wavelengths beyond 370 nm, for the purpose of controlling photo-induced pigmentation and its evolution by means of a system for screening out UV over the entire UV spectrum.

Among all the compounds that have been recommended for this purpose an advantageous family of UV-screening agents has been proposed, which consists of carbon-bearing merocyanine derivatives, which is described in

patent US 4 195 999, patent application WO 2004/006 878, patent applications WO2008/090066, WO2011/113718, WO2009/027258, WO2013010590, WO2013/011094, WO20130/11480 and the documents IP COM JOURNAL N°000179675D published on February 23, 2009, IP COM JOURNAL N°000182396D published on April 29, IP COM JOURNAL N° 000189542D published on November 12, 2009, IP COM Journal N°IPCOM000011179D published on 03/04/2004. Some of these compounds may show the following drawbacks :

- relatively unsatisfactory solubility in the usual solvents and in particular in fatty substances such as oils which may require a laborious formulation process and/or may result in cosmetic drawbacks such as a greasy effect on application ;
- an unsatisfactory chemical stability and/or unsatisfactory photostability
- produce a color liable to discourage the consumer from using a cosmetic or dermatological composition containing them.

Antisun compositions are quite often in the form of an emulsion of oil-in-water type (i.e. a cosmetically acceptable support consisting of a continuous aqueous dispersing phase and of a discontinuous oily dispersed phase) or of the water-in-oil type (i.e. a cosmetically acceptable support consisting of a continuous oily dispersing phase and of a discontinuous aqueous dispersed phase) which contains, in varying concentrations, one or more conventional lipophilic and/or hydrophilic organic screening agents which are capable of selectively absorbing harmful UV rays, these screening agents (and the amounts thereof) being selected as a function of the desired sun protection factor.
The Applicant has found in the course of its research that some of the merocyanines could lose their efficacy in the presence of certain particular emulsifiers in antisun compositions. The Applicant has especially observed that some of these merocyanines degrade chemically in the presence of an emulsifying system containing at least one alkali metal salt of a phosphoric acid ester of a fatty alcohol.
There is thus still a need to select other families of emulsifiers which guarantee a good chemical stability of the merocyanines without the drawbacks as previously defined.
The Applicant has discovered, surprisingly, that the use of one emulsifying system containing at least one gemini surfactant and one merocyanine compound of formula (1) herein below make it possible to achieve this objective.
Furthermore, the merocyanine compounds of formula (1) herein below, present surprinsingly the advantage to be significantly less colored than the merocyanine compounds as disclosed in the application WO2008/090066 as the compound MC11 also called MC03 in the application WO2009/027258.
Those discoveries form the basis of the present invention.
Thus, in accordance with one of the objects of the present invention, a cosmetic or dermatological composition in emulsion form is now proposed, comprising, in a physiologically acceptable support:

a) at least one aqueous phase and
b) at least one oily phase, and
c) at least one merocyanine compound of formula (1) which will be defined in greater detail hereinbelow and
d) at least one emulsifying system containing at least one gemini surfactant.

Another subject of the present invention consists of a non-therapeutic cosmetic process for caring for and/or making up a keratin material, consisting in applying, to the surface of the said keratin material, at least one composition according to the invention as defined above.
The invention also relates to a non-therapeutic cosmetic process for limiting the darkening of the skin, improving the colour and uniformity of the complexion, comprising at least the application, to the surface of the keratin material, of at least one composition as defined previously.
The invention also relates to a non-therapeutic cosmetic process for treating the ageing of a keratin material, comprising at least the application, to the surface of the keratin material, of at least one composition as defined previously.
Other characteristics, aspects and advantages of the invention will emerge on reading the detailed description that follows.
The term "human keratin materials" means the skin (of the body, face and area around the eyes), hair, eyelashes, eyebrows, bodily hair, nails, lips or mucous membranes.
The term "physiologically acceptable" means compatible with the skin and/or its integuments, having a pleasant colour, odour and feel and not causing any unacceptable discomfort (stinging, tautness or redness) liable to discourage the consumer from using this composition.
The term "between X and Y" means the range of values also including the limits X and Y.
According to the invention, the term "preventing" or "prevention" means reducing the risk of occurrence or slowing down the occurrence of a given phenomenon, namely, according to the present invention, the signs of ageing of a keratin material.

The term "emulsion" means any macroscopically homogeneous, kinetically stable composition comprising at least two mutually immiscible phases; one being the dispersing continuous phase and the other being dispersed in the said continuous phase in the form of droplets. The two phases are kinetically stabilized by at least one emulsifying system generally comprising at least one emulsifying surfactant.

Emulsions are distinguished as being of the oil-in-water type, known as "direct" emulsions, consisting of an aqueous dispersing continuous phase and of an oily dispersed discontinuous phase, and emulsions of the water-in-oil type, known as inverse emulsions, consisting of an oily dispersing continuous phase and of an aqueous dispersed discontinuous phase. There are also multiple emulsions, for instance water-in-oil-in-water or oil-in-water-in-oil emulsions.

The term "emulsifying system" refers to any compound or mixture of compounds that is capable of increasing the kinetic stability of an emulsion. These compounds are generally amphiphilic and are surfactants characterized by their more or less hydrophilic or more or less lipophilic nature which will determine their ability to stabilize direct emulsions or inverse emulsions. They are especially classified by their HLB according to the calculation method of W.C. Griffin in the document "Classification of Surface Active Agents by HLB, Journal of the Society of Cosmetic Chemists 1 (1949) 311" and in the document "Calculation of HLB of Non Ionic Surfactants, Journal of the Society of Cosmetic Chemists 5 (1954) 249". The calculation of the HLB according to this calculation method is performed according to the equation:

$$HLB = 20 \text{ X } Mh/M$$

where Mh is the molar mass of the hydrophilic part of the surfactant and M is the total molecular mass of the molecule.

The term "gemini surfactant" means any dimeric molecule bearing two surfactant units each consisting of a hydrophilic head and a hydrophobic tail and connected together, at the hydrophilic heads, via a spacer group. For a detailed description of the various chemical structures and of their physicochemical properties, reference may be made to the following publications:

Milton J. Rosen, Gemini Surfactants, Properties of surfactant molecules with two hydrophilic groups and two hydrophobic groups, Cosmetics & Toiletries Magazine, vol. 113, December 1998, pp 49-55,

Milton J. Rosen, Recent Developments in Gemini Surfactants, Allured's Cosmetics & Toiletries Magazine, July 2001, vol. 116, No. 7, pp 67-70.

## MEROCYANINES

According to the present invention, the merocyanine compounds in accordance with the invention correspond to formula (1) below, and also the E/E- or E/Z-geometrical isomer forms thereof:

(1) 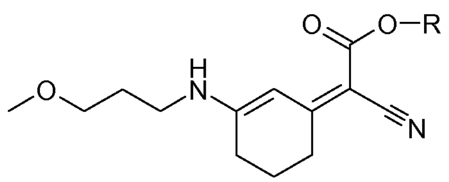

in which:

R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O.

The merocyanine compounds of the invention may be in their E/E- or E/Z-geometrical isomer forms.

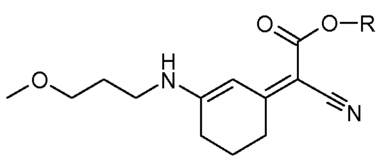

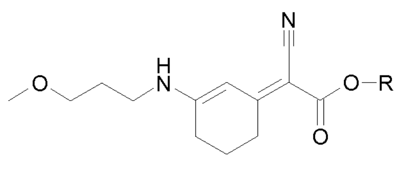

The preferential compounds of formula (1) are those in which:

R is a $C_1$-$C_{22}$ alkyl, which may be interrupted with one or more O.

Among the compounds of formula (1), use will be made more particularly of those chosen from the following compounds, and also the E/E- or E/Z- geometrical isomer forms thereof:

| | | | |
|---|---|---|---|
| 1 | 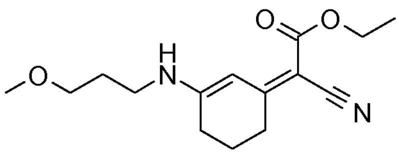<br>ethyl (2Z)-cyano{3-[(3-methoxypropyl)amino] cyclohex-2-en-1-ylidene}ethanoate | 4 | 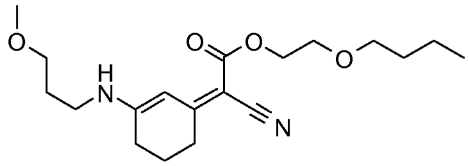<br>2-butoxyethyl (2Z)-cyano{3-[(3-methoxypropyl) amino]cyclohex-2-en-1-ylidene}ethanoate |
| 2 | 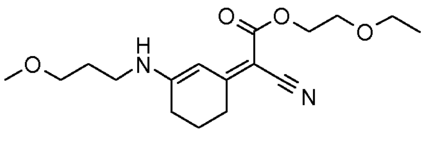<br>2-ethoxyethyl (2Z)-cyano{3-[(3-methoxypropyl) amino]cyclohex-2-en-1-ylidene}ethanoate | 5 | 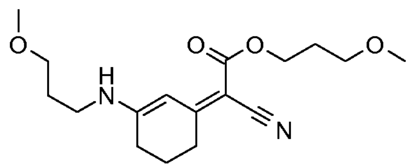<br>3-methoxypropyl (2Z)-cyano{3-[(3-methoxypropyl) amino ]cyclohex-2-en-1-ylidene}ethanoate |
| 3 | 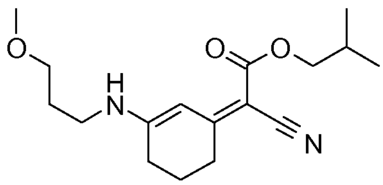<br>2-methylpropyl (2Z)-cyano{3-[(3-methoxypropyl) amino]cyclohex-2-en-1-ylidene}ethanoate | 6 | 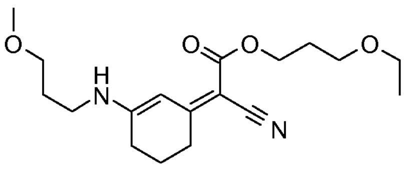<br>3-ethoxypropyl (2Z)-cyano{3-[(3-methoxypropyl) amino]cyclohex-2-en-1-ylidene}ethanoate |

According to a more particularly preferred mode of the invention, use will be made of the compound 2-ethoxyethyl (2Z)-cyano{3-[(3-methoxypropyl)amino]cyclohex-2-en-1-ylidene}ethanoate (2) in its E/Z geometrical configuration having the following structure:

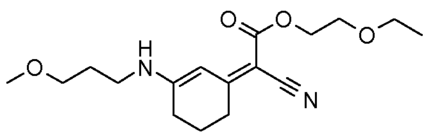

and/or in its E/E geometrical configuration having the following structure:

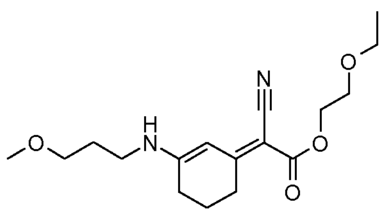

The merocyanines of formula (1) according to the invention are preferably present in the compositions according to the invention in a concentration ranging from 0.1% to 10% by weight and preferentially from 0.2% to 5% by weight relative to the total weight of the composition.

The compounds of formula (I) may be prepared according to the protocols described in Pat. Appl. WO 2007/071 582, in IP.com Journal (2009), 9(5A), 29-30 IPCOM000182396D under the title "Process for producing 3-amino-2-cyclohexan-1-ylidene compounds" and in US-A-4 749 643 on column 13, line 66 - column 14, line 57 and the references cited in this regard.

## EMULSIFYING SYSTEM

### a) Gemini surfactants

As preferred examples of gemini surfactants that may be used in the present invention, mention may be made of those collated in German patent application DE 199 43 681, namely the compounds of formula (I), described in WO 96/14926:

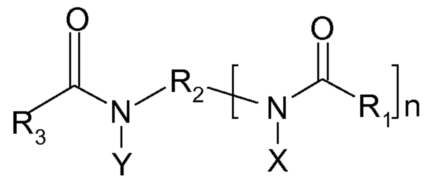

(I)

in which:

$R_1$ and $R_3$ denote, independently of each other, an alkyl radical containing from 1 to 25 carbon atoms;
$R_2$ denotes a spacer consisting of a linear or branched alkylene chain containing from 1 to 12 carbon atoms;
X denotes a group $-(C_2H_4O)_a-(C_3H_6O)_bZ$;
Y denotes a group $-(C_2H_4O)_c-(C_3H_6O)_dZ$;
in which
Z denotes a hydrogen atom or a radical $-CH_2-COOM$, $-SO_3M$, $-P(O)(OM)_2$, $-C_2H_4SO_3M$, $-C_3H_sSO_3M$ or $-CH_2(CHOH)_4CH_2OH$ group, in which M & M' represent H or an alkali metal or alkaline-earth metal or ammonium or alkanolammonium ion,
a and c, independently of each other, range from 0 to 15,
b and d, independently of each other, range from 0 to 10, and
the sum of a + b + c + d ranges from 1 to 25; and

- n ranges from 1 to 10.

$R_1$ and $R_3$ preferably denote, independently of each other, an alkyl radical containing from 5 to 21 and more particularly from 7 to 19 carbon atoms.
The gemini surfactant is preferably such that each of the groups $R_1$-CO- and $R_3$-CO- comprises from 8 to 20 carbon atoms and preferably denotes a coconut fatty acid residue (predominantly comprising lauric acid and myristic acid).
Preferably, b and d are equal to 0.
In addition, this surfactant is preferably such that the sum of a, b, c and d has a mean value ranging from 10 to 20 and is preferably from 12 to 18 and more particularly equal to 15.
A preferred group for Z is the group $-SO_3M$ in which M is preferably an alkali metal ion such as a sodium ion.
The spacer $R_2$ advantageously consists of a linear $C_1$-$C_3$ alkylene chain and preferably an ethylene chain ($-CH_2CH_2-$).
Finally, n is advantageously equal to 1.
A surfactant of this type is in particular the one identified by the INCI name: Disodium ethylene dicocamide PEG-15 disulfate, having the following structure:

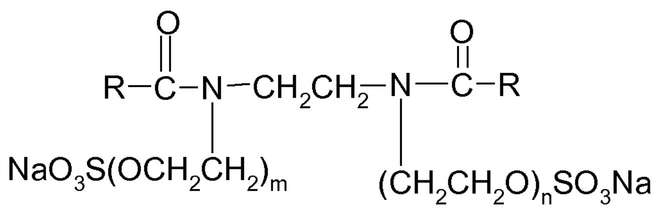

RCO represents a coconut fatty acid radical and m + n has a mean value of 15.
Preferably, the gemini surfactant according to the invention is used as a mixture with other surfactants and especially as a mixture with (a) glyceryl ester of a $C_6$-$C_{22}$ fatty acid (preferably $C_{14}$-$C_{20}$ such as a stearate), (b) a diester of a $C_6$-$C_{22}$ fatty acid (preferably $C_{14}$-$C_{20}$ such as a stearate) and of citric acid and of glycerol (especially a diester of a $C_6$-$C_{22}$ fatty acid and of glyceryl monocitrate), and (c) a $C_{10}$-$C_{30}$ fatty alcohol (preferably behenyl alcohol).

Advantageously, the composition according to the invention comprises a mixture of disodium ethylene dicocamide PEG-15 disulfate, glyceryl stearate, glyceryl stearate monocitrate and behenyl alcohol.

More preferentially, the gemini surfactant according to the invention represents from 10% to 20% by weight and advantageously 15% by weight; the glyceryl ester of a $C_6$-$C_{22}$ fatty acid represents from 30% to 40% by weight, advantageously 35% by weight; the diester of a $C_6$-$C_{22}$ fatty acid and of citric acid and of glycerol represents from 10% to 20% by weight, advantageously 15% by weight; and the $C_{10}$-$C_{30}$ fatty alcohol represents from 30% to 40% by weight, advantageously 35% by weight, relative to the total weight of the mixture of surfactants comprising the gemini surfactant.

Advantageously, the composition according to the invention comprises a mixture of from 10% to 20% by weight of disodium ethylene dicocamide PEG-15 disulfate, from 30% to 40% (in particular 35%) by weight of glyceryl stearate, from 10% to 20% (in particular 15%) by weight of glyceryl stearate monocitrate, and from 30% to 40% (in particular 35%) by weight of behenyl alcohol, relative to the total weight of the mixture of surfactants comprising the gemini surfactant.

As a variant, the gemini surfactant according to the invention may be used as a mixture with an anionic surfactant, such as an ester of lauric acid, sodium lauroyl lactate. In this case, the gemini surfactant preferably represents from 30% to 50% by weight and the anionic surfactant represents from 50% to 70% by weight, relative to the total weight of the mixture.

The gemini surfactant may be used, for example, as a mixture with other surfactants in the form of the products sold by Sasol under the Ceralution® names, in particular the following products:

- Ceralution® H: Behenyl Alcohol, Glyceryl Stearate, Glyceryl Stearate Citrate and Disodium Ethylene Dicocamide PEG-15 Disulfate,
- Ceralution® F: Sodium Lauroyl Lactylate and Disodium Ethylene Dicocamide PEG-15 Disulfate,
- Ceralution® C: Capric/Caprylic Triglyceride, Ceteareth-25, Disodium Ethylene Dicocamide PEG-15 Disulfate, Sodium Lauroyl Lactylate, Behenyl Alcohol, Glyceryl Stearate and Glyceryl Stearate Citrate (INCI names).

The gemini surfactant represents from 3% to 50% of the weight of these mixtures.

The gemini surfactant may be present in the composition according to the invention in an active material content ranging from 0.05% to 10% by weight, preferably ranging from 0.1% to 5% by weight and better still ranging from 0.2% to 2% by weight relative to the total weight of the composition.

## OILY PHASE

The compositions in accordance with the invention comprise at least one oily phase.

For the purposes of the invention, the term "oily phase" means a phase comprising at least one oil and all of the liposoluble and lipophilic ingredients and the fatty substances used for the formulation of the compositions of the invention.

The term "oil" means any fatty substance which is in liquid form at room temperature (20 - 25°C) and at atmospheric pressure (760 mmHg).

An oil that is suitable for use in the invention may be volatile or non-volatile.

An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" means an oil comprising at least one silicon atom, and especially at least one Si-O group.

The term "hydrocarbon-based oil" means an oil containing mainly hydrogen and carbon atoms.

The term "fluoro oil" means an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase generally comprises, in addition to the lipophilic UV-screening agent(s), at least one volatile or non-volatile hydrocarbon-based oil and/or one volatile and/or non-volatile silicone oil.

For the purposes of the invention, the term "volatile oil" means an oil that is capable of evaporating on contact with the skin or the keratin fibre in less than one hour, at room temperature and atmospheric pressure. The volatile oil(s) of the invention are volatile cosmetic oils which are liquid at room temperature and which have a non-zero vapour pressure, at room temperature and atmospheric pressure, ranging in particular from 0.13 Pa to 40 000 Pa ($10^{-3}$ to 300 mmHg), in particular ranging from 1.3 Pa to 13 000 Pa (0.01 to 100 mmHg) and more particularly ranging from 1.3 Pa

to 1300 Pa (0.01 to 10 mmHg).

The term "non-volatile oil" means an oil which remains on the skin or the keratin fibre, at room temperature and atmospheric pressure, for at least several hours and which in particular has a vapour pressure of less than $10^{-3}$ mmHg (0.13 Pa).

Hydrocarbon-based oils

As non-volatile hydrocarbon-based oils that may be used according to the invention, mention may be made especially of:

(i) hydrocarbon-based oils of plant origin, such as glyceride triesters, which are generally triesters of fatty acids and of glycerol, the fatty acids of which can have varied chain lengths from $C_4$ to $C_{24}$, it being possible for these chains to be saturated or unsaturated and linear or branched; these oils are in particular wheat germ oil, sunflower oil, grape seed oil, sesame oil, corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin seed oil, marrow oil, blackcurrant oil, evening primrose oil, millet oil, barley oil, quinoa oil, rye oil, safflower oil, candlenut oil, passionflower oil and musk rose oil; or also caprylic/capric acid triglycerides, such as those sold by the company Stéarineries Dubois or those sold under the names Miglyol 810®, 812® and 818® by the company Dynamit Nobel;

(ii) synthetic ethers having from 10 to 40 carbon atoms;

(iii) linear or branched hydrocarbons of mineral or synthetic origin, such as petroleum jelly, polydecenes, hydrogenated polyisobutene such as Parleam, and squalane, and mixtures thereof;

(iv) synthetic esters, for instance oils of formula RCOOR' in which R represents a linear or branched fatty acid residue containing from 1 to 40 carbon atoms and R' represents a hydrocarbon-based chain that is especially branched, containing from 1 to 40 carbon atoms on condition that R + R' is ≥10, for instance Purcellin oil (cetearyl octanoate), isopropyl myristate, isopropyl palmitate, $C_{12}$-$C_{15}$ alkyl benzoate, such as the product sold under the trade name Finsolv TN® or Witconol TN® by the company Witco or Tegosoft TN® by the company Evonik Goldschmidt, 2-ethylphenyl benzoate, such as the commercial product sold under the name X-Tend 226® by the company ISP, isopropyl lanolate, hexyl laurate, diisopropyl adipate, isononyl isononanoate, oleyl erucate, 2-ethylhexyl palmitate, isostearyl isostearate, diisopropyl sebacate, such as the product sold under the name of "Dub Dis" by the company Stearinerie Dubois, octanoates, decanoates or ricinoleates of alcohols or polyalcohols, such as propylene glycol dioctanoate; hydroxylated esters, such as isostearyl lactate or diisostearyl malate; and pentaerythritol esters; citrates or tartrates, such as di(linear $C_{12}$-$C_{13}$ alkyl) tartrates, such as those sold under the name Cosmacol ETI® by the company Enichem Augusta Industriale, and also di(linear $C_{14}$-$C_{15}$ alkyl) tartrates, such as those sold under the name Cosmacol ETL® by the same company; or acetates;

(v) fatty alcohols that are liquid at room temperature, containing a branched and/or unsaturated carbon-based chain containing from 12 to 26 carbon atoms, for instance octyldodecanol, isostearyl alcohol, oleyl alcohol, 2-hexyldecanol, 2-butyloctanol or 2-undecylpentadecanol;

(vi) higher fatty acids, such as oleic acid, linoleic acid or linolenic acid;

(vii) carbonates such as dicaprylyl carbonate, for instance the product sold under the name Cetiol CC® by the company Cognis;

(viii) fatty amides, such as isopropyl N-lauroyl sarcosinate, such as the product sold under the trade name Eldew SL 205® from Ajinomoto;

and mixtures thereof.

Among the non-volatile hydrocarbon-based oils that may be used according to the invention, preference will be given more particularly to glyceride triesters and in particular to caprylic/capric acid triglycerides, synthetic esters and in particular isononyl isononanoate, oleyl erucate, $C_{12}$-$C_{15}$ alkyl benzoate, 2-ethylphenyl benzoate and fatty alcohols, in particular octyldodecanol.

As volatile hydrocarbon-based oils that may be used according to the invention, mention may be made especially of hydrocarbon-based oils containing from 8 to 16 carbon atoms and in particular of branched $C_8$-$C_{16}$ alkanes, such as $C_8$-$C_{16}$ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched $C_8$-$C_{16}$ esters, isohexyl neopentanoate, and mixtures thereof.

Mention may also be made of the alkanes described in the Cognis patent applications WO 2007/068 371 or WO 2008/155 059 (mixtures of distinct alkanes differing by at least one carbon). These alkanes are obtained from fatty alcohols, which are themselves obtained from coconut or palm oil. Mention may be made of the mixtures of n-undecane ($C_{11}$) and n-tridecane ($C_{13}$) obtained in Examples 1 and 2 of patent application WO 2008/155 059 from the company Cognis. Mention may also be made of n-dodecane ($C_{12}$) and n-tetradecane ($C_{14}$) sold by Sasol under the respective references Parafol 12-97 and Parafol 14-97®, and also mixtures thereof.

Other volatile hydrocarbon-based oils, for instance petroleum distillates, especially those sold under the name Shell Solt® by the company Shell, may also be used. According to one embodiment, the volatile solvent is chosen from volatile hydrocarbon-based oils containing from 8 to 16 carbon atoms, and mixtures thereof.

b) Silicone oils

The non-volatile silicone oils may be chosen in particular from non-volatile polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Examples of volatile silicone oils that may be mentioned include volatile linear or cyclic silicone oils, especially those with a viscosity $\leq$ 8 centistokes ($8 \times 10^{-6}$ $m^2/s$) and especially containing from 2 to 7 silicon atoms, these silicones optionally comprising alkyl or alkoxy groups containing from 1 to 10 carbon atoms. As volatile silicone oils that may be used in the invention, mention may be made especially of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Mention may also be made of the volatile linear alkyltrisiloxane oils of general formula (I):

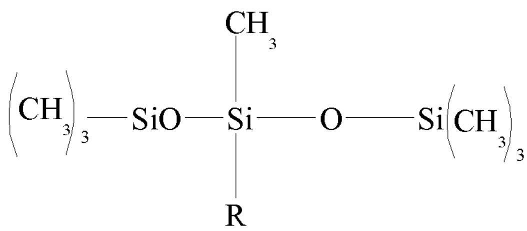

in which R represents an alkyl group comprising from 2 to 4 carbon atoms, of which one or more hydrogen atoms may be substituted with a fluorine or chlorine atom.

Among the oils of general formula (I) that may be mentioned are:

3-butyl-1,1,1,3,5,5,5-heptamethyltrisiloxane,
3-propyl-1,1,1,3,5,5,5-heptamethyltrisiloxane, and
3-ethyl-1,1,1,3,5,5,5-heptamethyltrisiloxane,
corresponding to the oils of formula (I) for which R is, respectively, a butyl group, a propyl group or an ethyl group.

Fluoro oils

Use may also be made of volatile fluoro oils, such as nonafluoromethoxybutane, decafluoropentane, tetradecafluorohexane, dodecafluoropentane, and mixtures thereof.

An oily phase according to the invention may also comprise other fatty substances, mixed with or dissolved in the oil.

Another fatty substance that may be present in the oily phase may be, for example:

- a fatty acid chosen from fatty acids comprising from 8 to 30 carbon atoms, such as stearic acid, lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;

- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

Preferentially, the overall oily phase, including all the lipophilic substances of the composition that are capable of being dissolved in this same phase, represents from 5% to 95% by weight and preferentially from 10% to 80% by weight, relative to the total weight of the composition.

## AQUEOUS PHASE

The compositions according to the invention comprise at least one aqueous phase.

The aqueous phase contains water and optionally other water-soluble or water-miscible organic solvents.

An aqueous phase that is suitable for use in the invention may comprise, for example, a water chosen from a natural spring water, such as water from La Roche-Posay, water from Vittel or waters from Vichy, or a floral water.

The water-soluble or water-miscible solvents that are suitable for use in the invention comprise short-chain monoalcohols, for example $C_1$-$C_4$ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.

According to a preferred embodiment, use may more particularly be made of ethanol, propylene glycol, glycerol, and mixtures thereof.

According to a particular form of the invention, the overall aqueous phase, including all the hydrophilic substances of the composition capable of being dissolved in this same phase, represents from 5% to 95% by weight and preferentially from 10% to 80% by weight, relative to the total weight of the composition.

## ADDITIVES

### a) Additional UV-screening agents:

The compositions according to the invention may also contain one or more additional UV-screening agents chosen from hydrophilic, lipophilic or insoluble organic UV-screening agents and/or one or more mineral pigments. It will preferentially consist of at least one hydrophilic, lipophilic or insoluble organic UV-screening agent.

The term "hydrophilic UV-screening agent" means any cosmetic or dermatological organic or mineral compound for screening out UV radiation, which can be fully dissolved in molecular form in a liquid aqueous phase or which can be dissolved in colloidal form (for example in micellar form) in a liquid aqueous phase.

The term "lipophilic screening agent" means any cosmetic or dermatological organic or mineral compound for screening out UV radiation, which can be fully dissolved in molecular form in a liquid fatty phase or which can be dissolved in colloidal form (for example in micellar form) in a liquid fatty phase.

The term "insoluble UV-screening agent" means any cosmetic or dermatological organic or mineral compound for screening out UV radiation which has a solubility in water of less than 0.5% by weight and a solubility of less than 0.5% by weight in the majority of organic solvents such as liquid paraffin, fatty alcohol benzoates and fatty acid triglycerides, for example Miglyol 812® sold by the company Dynamit Nobel. This solubility, determined at 70°C, is defined as the amount of product in solution in the solvent at equilibrium with an excess of solid in suspension after returning to room temperature. It may be readily evaluated in the laboratory.

The additional organic UV-screening agents are chosen in particular from cinnamic compounds; anthranilate compounds; salicylic compounds; dibenzoylmethane compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole derivatives; imidazoline compounds; bis-benzazolyl compounds, as described in patents EP 669 323 and US 2 463 264; p-aminobenzoic acid (PABA) compounds; methylenebis(hydroxyphenylbenzotriazole) compounds, as described in patent applications US 5 237 071, US 5 166 355, GB 2 303 549, DE 197 26 184 and EP 893 119; benzoxazole compounds, as described in patent applications EP 0 832 642, EP 1 027 883, EP 1 300 137 and DE 101 62 844; screening polymers and screening silicones, such as those described in particular in patent application WO 93/04665; α-alkylstyrene-based dimers, such as those described in patent application DE 198 55 649; 4,4-diarylbutadiene compounds, as described in patent applications EP 0 967 200, DE 197 46 654, DE 197 55 649, EP-A-1 008 586, EP 1 133 980 and EP 133 981, and mixtures thereof.

As examples of organic photoprotective agents, mention may be made of those denoted hereinbelow under their INCI name:

Cinnamic compounds:

Ethylhexyl Methoxycinnamate, sold in particular under the trade name Parsol MCX® by DSM Nutritional Products,
Isopropyl Methoxycinnamate,
Isoamyl p-methoxycinnamate sold under the trade name Neo Heliopan E 1000® by Symrise,
DEA Methoxycinnamate,
Diisopropyl Methyl Cinnamate,
Glyceryl Ethylhexanoate Dimethoxycinnamate.

para-Aminobenzoic compounds:

PABA,
Ethyl PABA,
Ethyl Dihydroxypropyl PABA,
Ethylhexyl Dimethyl PABA, sold in particular under the name Escalol 507® by ISP, Glyceryl PABA,
PEG-25 PABA, sold under the name Uvinul P 25® by BASF.

Salicylic compounds:

Homosalate, sold under the name Eusolex HMS® by Rona/EM Industries, Ethylhexyl Salicylate, sold under the name Neo Heliopan OS® by Symrise, Dipropylene Glycol Salicylate, sold under the name Dipsal® by Scher, TEA Salicylate, sold under the name Neo Heliopan TS® by Symrise.

β,β-Diphenylacrylate compounds:

Octocrylene, sold in particular under the trade name Uvinul N 539® by BASF,
Etocrylene, sold in particular under the trade name Uvinul N 35® by BASF.

Benzophenone compounds:

Benzophenone-1, sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF,
Benzophenone-5,
Benzophenone-6, sold under the trade name Helisorb 11® by Norquay,
Benzophenone-8, sold under the trade name Spectra-Sorb UV-24® by American Cyanamid,
Benzophenone-9, sold under the trade name Uvinul DS 49® by BASF,
Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by BASF,
1,1'-(1,4-Piperazinediyl)bis[1-[2-[4-(diethylamino)-2-hydroxybenzoyl]phenyl]methanone] (CAS 919803-06-8), such as described in patent application WO 2007/071 584; this compound advantageously being used in micronized form (mean size of 0.02 to 2 μm), which may be obtained, for example, according to the micronization process described in patent applications GB-A-2 303 549 and EP-A-893 119, and in particular in the form of an aqueous dispersion.

Benzvlidenecamphor compounds:

3-Benzylidene Camphor, manufactured under the name Mexoryl SD® by Chimex,
4-Methylbenzylidene Camphor, sold under the name Eusolex 6300® by Merck,
Benzylidene Camphor Sulfonic Acid, manufactured under the name Mexoryl SL® by Chimex,
Camphor Benzalkonium Methosulfate, manufactured under the name Mexoryl SO® by Chimex,
Terephthalylidene Dicamphor Sulfonic Acid, manufactured under the name Mexoryl SX® by Chimex,
Polyacrylamidomethyl Benzylidene Camphor, manufactured under the name Mexoryl SW® by Chimex.

Phenylbenzimidazole compounds:

Phenylbenzimidazole Sulfonic Acid, sold in particular under the trade name Eusolex 232® by Merck.

Bis-benzazolyl compounds:

Disodium Phenyl Dibenzimidazole Tetrasulfonate, sold under the trade name Neo Heliopan AP® by Haarmann and Reimer.

Phenylbenzotriazole compounds:

Drometrizole Trisiloxane, sold under the name Silatrizole® by Rhodia Chimie.

Methylenebis(hydroxyphenylbenzotriazole) compounds:

Methylene Bis-Benzotriazolyl Tetramethylbutylphenol, in particular in solid form, such as the product sold under the trade name Mixxim BB/100® by Fairmount Chemical, or in the form of an aqueous dispersion of micronized particles with a mean particle size ranging from 0.01 to 5 μm, more preferentially from 0.01 to 2 μm and more particularly from 0.020 to 2 μm, with at least one alkylpolyglycoside surfactant having the structure $C_nH_{2n+1}O(C_6H_{10}O_5)_xH$, in which n is an integer from 8 to 16 and x is the mean degree of polymerization of the $(C_6H_{10}O_5)$ unit and ranges from 1.4 to 1.6, as described in patent GB-A-2 303 549, sold in particular under the trade name Tinosorb M® by the company BASF, or in the form of an aqueous dispersion of micronized particles with a mean particle size ranging from 0.02 to 2 μm, more preferentially from 0.01 to 1.5 μm and more particularly from 0.02 to 1 μm, in the presence of at least one polyglyceryl mono($C_8$-$C_{20}$)alkyl ester with a degree of glycerol polymerization of at least 5, such as the aqueous dispersions described in patent application WO 2009/063 392.

Triazine compounds:

- Bis-Ethylhexyloxyphenol Methoxyphenyl Triazine, sold under the trade name Tinosorb S® by BASF,
- Ethylhexyl Triazone, sold in particular under the trade name Uvinul T150® by BASF,
- Diethylhexyl Butamido Triazone, sold under the trade name Uvasorb HEB® by Sigma 3V,
- 2,4,6-tris(dineopentyl 4'-aminobenzalmalonate)-s-triazine,
- 2,4,6-tris(diisobutyl 4'-aminobenzalmalonate)-s-triazine;
- 2,4-bis(n-butyl 4'-aminobenzoate)-6-(aminopropyltrisiloxane)-s-triazine,
- 2,4-bis(dineopentyl 4'-aminobenzalmalonate)-6-(n-butyl 4'-aminobenzoate)-s-triazine,
- symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC, West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 μm), which may be obtained, for example, according to the micronization process described in patent applications GB-A-2 303 549 and EP-A-893 119, and in particular in the form of an aqueous dispersion,
- silicone triazines substituted with two aminobenzoate groups, as described in patent EP 0 841 341, in particular 2,4-bis(n-butyl 4'-aminobenzalmalonate)-6-[(3-{1,3,3,3-tetramethyl-1-[(trimethylsilyl)oxy]disiloxanyl}propyl)amino]-s-triazine.

Anthranilic compounds:

Menthyl Anthranilate, sold under the trade name Neo Heliopan MA® by Symrise.

Imidazoline compounds:

Ethylhexyl dimethoxybenzylidene dioxoimidazoline propionate.

Benzalmalonate compounds:

Polyorganosiloxane comprising benzalmalonate functional groups, such as Polysilicone-15, sold under the trade name Parsol SLX® by Hoffmann-LaRoche.

4,4-Diarylbutadiene compounds:

1,1-Dicarboxy(2,2'-dimethylpropyl)-4,4-diphenylbutadiene.

Benzoxazole compounds:

2,4-Bis[4-[5-(1,1-dimethylpropyl)benzoxazol-2-yl]phenylimino]-6-[(2-ethylhexyl)imino]-1,3,5-triazine, sold under the name of Uvasorb K2A® by Sigma 3V.

The preferred organic screening agents are chosen from:

Ethylhexyl Methoxycinnamate,
Ethylhexyl salicylate,
Homosalate,
Octocrylene,
Phenylbenzimidazole Sulfonic Acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidene Dicamphor Sulfonic Acid,
Disodium Phenyl Dibenzimidazole Tetrasulfonate,
Methylene Bis-Benzotriazolyl Tetramethylbutylphenol,
Bis-Ethylhexyloxyphenol Methoxyphenyl Triazine,
Ethylhexyl Triazone,
Diethylhexyl Butamido Triazone,
2,4,6-Tris(dineopentyl 4'-aminobenzalmalonate)-s-triazine,
2,4,6-Tris(diisobutyl 4'-aminobenzalmalonate)-s-triazine,
2,4-Bis(n-butyl 4'-aminobenzoate)-6-(aminopropyltrisiloxane)-s-triazine,
2,4-Bis(dineopentyl 4'-aminobenzalmalonate)-6-(n-butyl 4'-aminobenzoate)-s-triazine,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
2,4,6-Tris(diphenyl)triazine,
2,4,6-Tris(terphenyl)triazine,
Drometrizole Trisiloxane,
Polysilicone-15,
1,1-Dicarboxy(2,2'-dimethylpropyl)-4,4-diphenylbutadiene,
2,4-Bis[4-[5-(1,1-dimethylpropyl)benzoxazol-2-yl]phenylimino]-6-[(2-ethylhexyl)imino]-1,3,5-triazine,
and mixtures thereof.

The particularly preferred organic screening agents are chosen from:

Ethylhexyl salicylate,
Homosalate,
Octocrylene,

n-Hexyl 2-(4-diethylamino-2-hydroxybenzoyl)benzoate,
Terephthalylidene Dicamphor Sulfonic Acid,
Bis(ethylhexyloxyphenol)methoxyphenyltriazine,
Ethylhexyl Triazone,
Diethylhexyl Butamido Triazone,
2,4-Bis(n-butyl 4'-aminobenzalmalonate)-6-[(3-{1,3,3,3-tetramethyl-1-[(trimethylsilyl)oxy]disiloxanyl}propyl)amino]-s-triazine,
Drometrizole Trisiloxane,
and mixtures thereof.

The mineral UV-screening agents used in accordance with the present invention are metal oxide pigments. More preferentially, the mineral UV-screening agents are metal oxide particles with a mean elementary particle size of less than or equal to 0.5 $\mu$m, more preferentially between 0.005 and 0.5 $\mu$m, even more preferentially between 0.01 and 0.2 $\mu$m, better still between 0.01 and 0.1 $\mu$m and more particularly between 0.015 and 0.05 $\mu$m.

They may be selected in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:

- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100 SA from the company Tayca and Tioveil from the company Tioxide,
- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
- with silica, alumina and alginic acid, such as the product MT-100 AQ® from the company Tayca,
- with alumina and aluminium laurate, such as the product Microtitanium Dioxide MT 100 S® from the company Tayca,
- with iron oxide and iron stearate, such as the product Microtitanium Dioxide MT 100 F® from the company Tayca,
- with zinc oxide and zinc stearate, such as the product BR 351® from the company Tayca,
- with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
- with silica, alumina and aluminium stearate and treated with a silicone, such as the product STT-30-DS® from the company Titan Kogyo,
- with silica and treated with a silicone, such as the product UV-Titan X 195® from the company Sachtleben Pigments,
- with alumina and treated with a silicone, such as the products Tipaque TTO-55 (S)® from the company Ishihara or UV Titan M 262® from the company Sachtleben Pigments,
- with triethanolamine, such as the product STT-65-S from the company Titan Kogyo,
- with stearic acid, such as the product Tipaque TTO-55 (C)® from the company Ishihara,
- with sodium hexametaphosphate, such as the product Microtitanium Dioxide MT 150 W® from the company Tayca,
- $TiO_2$ treated with octyltrimethylsilane, sold under the trade name T 805® by the company Degussa Silices,
- $TiO_2$ treated with a polydimethylsiloxane, sold under the trade name 70250 Cardre UF TiO2SI3® by the company Cardre,
- anatase/rutile $TiO_2$ treated with a polydimethylhydrosiloxane, sold under the trade name Microtitanium Dioxide USP Grade Hydrophobic® by the company Color Techniques.

Mention may also be made of $TiO_2$ pigments doped with at least one transition metal such as iron, zinc or manganese and more particularly manganese. Preferably, the said doped pigments are in the form of an oily dispersion.

The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of $TiO_2$ particles doped with manganese in capric/caprylic acid triglycerides in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50® by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackher under the name Transparent Titanium Oxide PW®, by the company Miyoshi Kasei under the name UFTR®, by the company Tomen under the name ITS® and by the company Tioxide under the name Tioveil AQ®.

The uncoated zinc oxide pigments are for example:

- those sold under the name Z-Cote by the company Sunsmart;
- those sold under the name Nanox® by the company Elementis;
- those sold under the name Nanogard WCD 2025® by the company Nanophase Technologies.

The coated zinc oxide pigments are for example:

- those sold under the name Zinc Oxide CS-5® by the company Toshibi (ZnO coated with polymethylhydrogenosiloxane);
- those sold under the name Nanogard Zinc Oxide FN® by the company Nanophase Technologies (as a 40% dispersion in Finsolv TN®, $C_{12}$-$C_{15}$ alkyl benzoate);
- those sold under the name Daitopersion Zn-30® and Daitopersion Zn-50® by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);
- those sold under the name SPD-Z1® by the company Shin-Etsu (ZnO coated with silicone-grafted acrylic polymer, dispersed in cyclodimethylsiloxane);
- those sold under the name Escalol Z100® by the company ISP (alumina-treated ZnO dispersed in the ethylhexyl methoxycinnamate/PVP-hexadecene copolymer/methicone mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in $C_{12}$-$C_{15}$ alkyl benzoate with hydroxystearic acid polycondensate).

The uncoated cerium oxide pigments may be, for example, those sold under the name Colloidal Cerium Oxide® by the company Rhone-Poulenc.

The uncoated iron oxide pigments are sold, for example, by the company Arnaud under the names Nanogard WCD 2002® (FE 45B®), Nanogard Iron FE 45 BL AQ®, Nanogard FE 45R AQ® and Nanogard WCD 2006® (FE 45R®) or by the company Mitsubishi under the name TY-220®.

The coated iron oxide pigments are sold, for example, by the company Arnaud under the names Nanogard WCD 2008 (FE 45B FN)®, Nanogard WCD 2009® (FE 45B 556®), Nanogard FE 45 BL 345® and Nanogard FE 45 BL® or by the company BASF under the name Transparent Iron Oxide®.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 261® sold by the company Sachtleben Pigments, or coated with alumina, silica and glycerol, such as the product M 211® sold by the company Sachtleben Pigments.

According to the invention, coated or uncoated titanium oxide pigments are particularly preferred.

The additional UV-screening agents according to the invention are preferably present in the compositions according to the invention in a content ranging from 0.1% to 45% by weight and in particular from 5% to 30% by weight relative to the total weight of the composition.

**b) Other additives:**

The aqueous compositions in accordance with the present invention may also comprise conventional cosmetic adjuvants chosen in particular from organic solvents, ionic or nonionic thickeners, softeners, humectants, opacifiers, stabilizers, emollients, silicones, antifoams, fragrances, preserving agents, anionic, cationic, nonionic, zwitterionic or amphoteric surfactants, active agents, fillers, polymers, propellants, basifying or acidifying agents or any other ingredient commonly used in the cosmetic and/or dermatological field.

Among the organic solvents that may be mentioned are lower alcohols and polyols. These polyols may be chosen from glycols and glycol ethers, for instance ethylene glycol, propylene glycol, butylene glycol, dipropylene glycol or diethylene glycol.

Mention may be made, as thickeners, of carboxyvinyl polymers, such as the Carbopols (Carbomers) and the Pemulens (acrylate/$C_{10}$-$C_{30}$ alkyl acrylate copolymer) (Pemulen TR1® or Pemulen TR2®); polyacrylamides, such as, for example, the crosslinked copolymers sold under the names Sepigel 305® (CTFA name: polyacrylamide/$C_{13-14}$ isoparaffin/Laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, such as the poly(2-acrylamido-2-methylpropanesulfonic acid) sold by the company Hoechst under the trade name Hostacerin AMPS® (CTFA name: ammonium polyacryloyldimethyl taurate) or Simulgel 800®, sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, such as Simulgel NS® and Sepinov EMT 10®, sold by the company SEPPIC; cellulose derivatives, such as hydroxyethylcellulose; polysaccharides and in particular gums, such as xanthan gum; water-soluble or water-dispersible silicone derivatives, such as acrylic silicones, polyether silicones and cationic silicones, and mixtures thereof.

Among the acidifying agents, examples that may be mentioned include mineral or organic acids, for instance hydrochloric acid, orthophosphoric acid, sulfuric acid, carboxylic acids, for instance acetic acid, tartaric acid, citric acid or lactic acid, and sulfonic acids.

Among the basifying agents, examples that may be mentioned include aqueous ammonia, alkali metal carbonates, alkanolamines, such as mono-, di- and triethanolamines and derivatives thereof, sodium hydroxide or potassium hydroxide.

Preferably, the cosmetic composition comprises one or more basifying agents selected from alkanolamines, in particular triethanolamine, and sodium hydroxide.

In the case of a direct emulsion, the pH of the composition in accordance with the invention is generally between 3 and 12 approximately, preferably between 5 and 11 approximately and even more particularly from 6 to 8.5.

Among the active agents for caring for keratin materials such as the skin, the lips, the scalp, the hair, the eyelashes or the nails, examples that may be mentioned include:

- vitamins and derivatives or precursors thereof, alone or as mixtures;
- antioxidants;
- free-radical scavengers;
- antipollution agents;
- self-tanning agents;
- antiglycation agents;
- calmatives;
- deodorants;
- essential oils;
- NO-synthase inhibitors;
- agents for stimulating the synthesis of dermal or epidermal macromolecules and/or for preventing their degradation;
- agents for stimulating fibroblast proliferation;
- agents for stimulating keratinocyte proliferation;
- muscle relaxants,
- refreshing agents;
- tensioning agents;
- mattifying agents;
- depigmenting agents;
- propigmenting agents;
- keratolytic agents;
- desquamating agents;
- moisturizers;
- antiinflammatory agents;

- antimicrobial agents;
- slimming agents;
- agents acting on the energy metabolism of cells;
- insect repellents;
- substance P or CGRP antagonists;
- hair-loss counteractants;
- antiwrinkle agents;
- antiageing agents.

A person skilled in the art will select the said active principle(s) according to the effect desired on the skin, the hair, the eyelashes, the eyebrows or the nails.

Needless to say, a person skilled in the art will take care to select the abovementioned optional additional compound(s) and/or the amounts thereof such that the advantageous properties intrinsically associated with the compositions in accordance with the invention are not, or are not substantially, adversely affected by the envisaged addition(s).

## GALENICAL FORMS

The compositions according to the invention may be prepared according to the techniques that are well known to those skilled in the art for manufacturing emulsions, and in particular oil-in-water emulsions. They may be in the form of a cream, a milk or a cream gel. They may optionally be in the form of a mousse or a spray.

The compositions according to the invention are preferably in the form of an oil-in-water emulsion.

The emulsification processes that may be used are of the paddle or impeller, rotor-stator and HPH type.

In order to obtain stable emulsions with a low content of polymer (oil/polymer ratio > 25), it is possible to prepare the dispersion in concentrated phase and then to dilute the dispersion with the remainder of the aqueous phase.

It is also possible, by means of an HPH (between 50 and 800 bar), to obtain stable dispersions with droplet sizes that may be as low as 100 nm.

The aqueous phase of the emulsions may comprise a nonionic vesicular dispersion prepared according to known processes (Bangham, Standish and Watkins, J. Mol. Biol. 13, 238 (1965), FR 2 315 991 and FR 2 416 008).

The compositions according to the invention find their application in a large number of treatments, especially cosmetic treatments, of the skin, the lips and the hair, including the scalp, especially for protecting and/or caring for the skin, the lips and/or the hair, and/or for making up the skin and/or the lips.

Another subject of the present invention consists of the use of the compositions according to the invention as defined above for the manufacture of cosmetic products for treating the skin, the lips, the nails, the hair, the eyelashes, the eyebrows and/or the scalp, especially care products, antisun products and makeup products.

The cosmetic compositions according to the invention may be used, for example, as makeup products.

Another subject of the present invention consists of a non-therapeutic cosmetic process for caring for and/or making up a keratin material, which consists in applying, to the surface of the said keratin material, at least one composition according to the invention as defined above.

The cosmetic compositions according to the invention may be used, for example, as care products and/or antisun products for the face and/or body with a liquid to semi-liquid consistency, such as milks, more or less smooth creams, cream gels or pastes. They may optionally be packaged in an aerosol and be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged as an aerosol in accordance with the invention generally comprise conventional propellants, such as, for example, hydrofluoro compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

## ASSEMBLY

According to another aspect, the invention also relates to a cosmetic assembly comprising:

i) a container delimiting one or more compartment(s), the said container being closed by a closing member and optionally not being leaktight; and

ii) a makeup and/or care composition in accordance with the invention placed inside the said compartment(s).

The container may be, for example, in the form of a jar or a box.

The closing member may be in the form of a lid comprising a cap mounted so as to be able to move by translation or by pivoting relative to the container housing the said makeup and/or care composition(s).

The examples that follow serve to illustrate the invention without, however, being limiting in nature. In these examples, the amounts of the composition ingredients are given as weight percentages relative to the total weight of the composition.

**Example A1: Preparation of compound (1)**

(1)

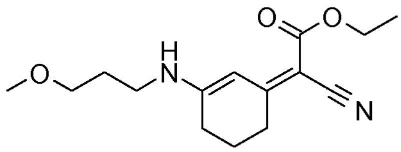

122.23 g of 3-[(3-methoxypropyl)amino]-2-cyclohexen-1-one were alkylated with dimethyl sulfate or alternatively with diethyl sulfate and treated with 75.45 g of ethyl cyanoacetate in approximately equimolar proportions in the presence of a base and optionally of a solvent. The following base/solvent combinations were used:

| Example | Base | Solvent |
|---|---|---|
| Example A1.1 | DBU (1,8-diazabicyclo[5.4.0]undec-7-ene) | dimethylacetamide |
| Example A1.2 | triethylamine | isopropanol |
| Example A1.3 | 3-methoxypropylamine | isopropanol |
| Example A1.4 | 3-methoxypropylamine | tert-amyl alcohol |
| Example A1.5 | 3-methoxypropylamine | toluene |
| Example A1.6 | 3-methoxypropylamine | dimethylformamide |
| Example A1.7 | 3-methoxypropylamine | no solvent |
| Example A1.8 | N-morpholine | isopropanol |

The completion of the alkylation reaction was monitored, for example, via methods such as TLC, GC or HPLC. 162.30 g of compound (14) were obtained in the form of a brown oil. After crystallization, the product was obtained in the form of yellowish crystals. Melting point: 92.7°C

**Example A2: Preparation of compound (2)**

(2)

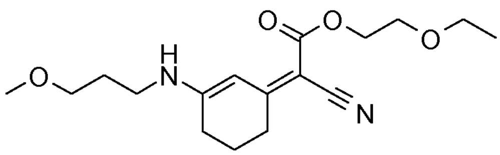

148.4 g of 3-[(3-methoxypropyl)amino]-2-cyclohexen-1-one were alkylated with dimethyl sulfate or alternatively with diethyl sulfate and treated with 130.00 g of 2-ethoxyethyl cyanoacetate in the presence of an organic base and of a solvent. The following base/solvent combinations were used:

| Example | Base | Solvent |
|---|---|---|
| Example A2.1 | DBU (1,8-diazabicyclo[5.4.0]undec-7-ene) | dimethylacetamide |
| Example A2.2 | triethylamine | isopropanol |

(continued)

| Example | Base | Solvent |
|---|---|---|
| Example A2.3 | 3-methoxypropylamine | isopropanol |
| Example A2.4 | N-methylmorpholine | tert-amyl alcohol |
| Example A2.5 | 3-methoxypropylamine | toluene |
| Example A2.6 | 3-methoxypropylamine | dimethylformamide |
| Example A2.7 | 3-methoxypropylamine | no solvent |

**Formulation examples 1 to 6**

The formulations 1 to 4 below were prepared, in which the chemical stability of compound (2) of the invention was evaluated.

| Phase | Ingredients | Formulation 1 (invention) | Formulation 2 (outside the invention) |
|---|---|---|---|
| A | AQUA | qsp 100 | qsp 100 |
| | DISODIUM EDTA | 0.1 | 0.1 |
| | GLYCEROL | 4 | 5 |
| | PROPYLENE GLYCOL | 4 | - |
| | PRESERVING AGENTS | 0.3 | - |
| | TRIETHANOLAMINE | - | 0.45 |
| | POTASSIUM CETYL PHOSPHATE (AMPHISOL K® -DSM NUTRITIONAL PRODUCTS) | | 1 |
| B | BEHENYL ALCOHOL (and) GLYCEROYL STEARATE (and) DISODIUM ETHYLENE DICOCAMIDE PEG-15 DISULFATE 15% active material (and) GLYCERYL STEARATE CITRATE (CERALUTION® H) | 2 | - |
| | CETYL ALCOHOL | 0.5 | 0.5 |
| | STEARIC ACID | - | 1.5 |
| | GLYCERYL STEARATE (and) PEG-100 STEARATE (ARLACEL 165®) | - | 2.5 |
| | CETEARYL ALCOHOL (and) CETEARYL GLUCOSIDE (MONTANOV 68® - SEPPIC) | - | 2 |
| | ISONONYL ISONONANOATE | 2 | - |
| | C12-15 ALKYL BENZOATE | 16.69 | - |
| | DIISOPROPYL SEBACATE | 4 | - |
| | PHENETHYL BENZOATE (and) BENZOIC ACID (X-TEND 226® - ISP) | | 30 |
| | COMPOUND 2 | 0.5 | 0.5 |
| | CYCLOHEXASILOXANE | 2 | - |
| | DIMETHICONE | - | 0.5 |
| | TOCOPHEROL | 0.1 | - |
| | PRESERVING AGENTS | - | 1 |

(continued)

| Phase | Ingredients | Formulation 1 (invention) | Formulation 2 (outside the invention) |
|---|---|---|---|
| C | AMMONIUM POLYACRYLDIMETHYLTAURAMIDE (HOSTACERIN AMPS®- CLARIANT) | 0.5 | - |
| | ACRYLATES COPOLYMER (CARBOPOL AQUA SF-1 POLYMER ®- LUBRIZOL) | 2 | - |
| | ISOHEXADECANE | - | 1 |
| | XANTHAN GUM | - | 0.2 |
| | ACRYLATES/$C_{10-30}$ ALKYL ACRYLATE CROSSPOLYMER (PEMULEN TR1) | - | 0.2 |
| D | TRIETHANOLAMINE | 0.4 | 0.2 |
| | ALCOHOL | 7 | - |

| Phase | Ingredients | Formule 3 (outside the invention) | Formule 4 (invention) |
|---|---|---|---|
| A | Water | qsp 100 | qsp 100 |
| | Glycerin | 5 | 5 |
| | Disodium EDTA | 0.1 | 0.1 |
| | Potassium Cetyl Phosphate (Amphisol K®) | 1 | - |
| B | Isopropyl Lauroyl Sarcosinate (Eldew SL-205®) | 30 | 30 |
| | Compound (2) | 2 | 2 |
| | Cetyl Alcohol | 0.5 | 0.5 |
| | Cetearyl Alcohol (and) Cetearyl Glucoside (Montanov 68®) | 2 | 2 |
| | Behenyl Alcohol (and) Glyceryl Stearate (and) disodium Ethylene Dicocamide PEG-15 Disulfate (and) Glyceryl Stearate Citrate (Céralution® H) | - | 1 |
| | Preservatives | 0.9 | 0.9 |
| C | Isohexadecane | 1 | 1 |
| | Acrylates/C10-C30 Alkyl Acrylate Crosspolymer (Pemulen TR1) | 0.2 | 0.2 |
| | Xanthan gum | 0.2 | 0.2 |
| D | Water | 2 | 2 |
| | Triethanolamine | 0.2 | 0.2 |
| E | Ethanol | 6 | 6 |
| **% compound (2) after 1 week at 60°C** | | 1.81 | 1.97 |
| **% loss of compound (2) after 1 week at 60°C relative to the theoretical content** | | **9,5** | **1,5** |

Formulations 5 and 6 below were prepared, in which the color was evaluated.

| Phase | Ingredients | Formule 5 (invention) | Formule 6 (hors invention) |
|---|---|---|---|
| A | Water | qsp 100 | qsp 100 |
| | Glycerin | 5 | 5 |
| | Disodium EDTA | 0.1 | 0.1 |
| B | Isopropyl Lauroyl Sarcosinate (Eldew SL-205®) | 30 | 30 |
| | Compound (2) | 2 | - |
| | MC11 of WO2008/090066 | - | 2 |
| | Cetyl Alcohol | 0.5 | 0.5 |
| | Cetearyl Alcohol (and) Cetearyl Glucoside (Montanov 68®) | 2 | 2 |
| | Behenyl Alcohol (and) Glyceryl Stearate (and) disodium Ethylene Dicocamide PEG-15 Disulfate (and) Glyceryl Stearate Citrate (Céralution® H) | **1** | **1** |
| | Preservatives | 0,9 | 0,9 |
| C | Isohexadecane | 1 | 1 |
| | Acrylates/C10-C30 Alkyl Acrylate Crosspolymer (Pemulen TR1) | 0.2 | 0.2 |
| | Xanthan gum | 0.2 | 0.2 |
| D | Water | 2 | 2 |
| | Triethanolamine | 0.2 | 0.2 |
| E | Ethanol | 6 | 6 |

**Emulsion preparation method:**

The aqueous phase A and oily phase B were prepared by mixing the starting materials with mechanical stirring at 80°C. Once the aqueous solution A and oily solution B were macroscopically homogeneous, the emulsion was prepared by introducing phase B into phase A with stirring using a rotor-stator homogenizer (mixer in the case of the invention). Stirring was continued for 10 to 20 minutes before adding phase C, with continued stirring. The emulsion was cooled to room temperature before adding, one by one, the ingredients of phase D.

**Protocol for evaluating the merocyanine stability:**

The stability of the merocyanines in formulation was evaluated by UPLC assay of the residual merocyanine content after 2 months of storage of the formulations at 4°C, at room temperature and at 45°C or after 1 week at 60)C. The percentage of degradation of the merocyanines after 2 months at 45°C or 1 week at 60 °C is expressed as:

$$\text{Degradation}_{t2M45°C}(\%) = \frac{\text{Ct Merocyanine}_{t2M45°C} - \text{Ct Merocyanine}_{t2MRT°C \text{ or } 4°C}}{\text{Ct Merocyanine}_{t2MRT°C \text{ or } 4°}} \times 100$$

$$\text{Degradation}_{t1W60°C}(\%) = \frac{\text{Ct Merocyanine}_{t1W60°C} - \text{Ct Merocyanine}_{t0}}{\text{Ct Merocyanine}_{t0}} \times 100$$

$t_0$ corresponds to the initial content introduced in the formulation.

| Chemical stability evaluated | Formulation 1 (invention) | Formulation 2 (outside the invention) |
|---|---|---|
| Residual merocyanine after 2 months at 45°C | 0.50 | 0.46 |

(continued)

| Chemical stability evaluated | Formulation 1 (invention) | Formulation 2 (outside the invention) |
|---|---|---|
| Merocyanine degradation after 2 months at 45°C (%) versus at t = 0 | <1% | 8% |

| Chemical stability evaluated | Formulation 3 (invention) | Formulation 4 (outside the invention) |
|---|---|---|
| Residual merocyanine after 1 week at 60°C (%) | 1.81 | 1.97 |
| Merocyanine degradation after 1 week at 60°C (%) versus at t = 0 | **9.5** | **1.5** |

The stability results obtained on formulation 1 according to the invention in comparison with formulation 2, or on formulation 3 according to the invention in comparison with formulation 4 show that the merocyanines of the invention, formulated in emulsified compositions of the invention in the presence of a gemini surfactant, are chemically more stable than when they are formulated in a support comprising an emulsifying system containing at least one alkali metal salt of a fatty alkyl phosphoric acid ester.

**Protocol for evaluating the color of the formulations**

The color of the formulations was evaluated after preparation of thin films on contrast map. The formulations were deposited within a circle of 2.2 cm of diameter and planed to obtain thicknesses of reproducible deposit. The colorimetric measures were then made by means of a spectro-colorimeter Minolta CM2600D in two points of the film. This operation is twice reproduced, which leads to 4 experimental values by formulation.

The results are expressed in the system (L *, has *, b *) in which L* represents the luminance, a* represents the red-green axis (-a* = green, +a* = red) and b* represents the yellow-blue axis (-b* blue, +b* yellow). So, a* and b* express the shade of the compound.

The difference of color ΔE was calculated from the variations ΔL*, Δa* et Δb* between the compound (2) and the compound MC11 with the following equation :

$$(\Delta E^*)^2 = (\Delta L^*)^2 + (\Delta a^*)^2 + (\Delta b^*)^2$$

$$\Delta L^* = L^*_{\text{formulation with compound MC11}} - L^*_{\text{formulation with compound (2)}}$$

$$\Delta a^* = a^*_{\text{formulation with compound MC11}} - a^*_{\text{formulation with compound (2)}}$$

$$\Delta b^* = b^*_{\text{formulation with compound MC11}} - b^*_{\text{formulation with compound (2)}}$$

We consider that the difference of color between the two compounds is significant if ΔE > 2.

**Colorimetric measures on the formulations 5 and 6**

| | Formule 5 (invention) | Formule 6 (outside the invention) |
|---|---|---|
| .L* | 92,9 ± 0.6 | 92.6 ± 0.6 |
| a* | -4.8 ± 0.6 | -5.9 ± 0.1 |

(continued)

| | Formule 5 (invention) | Formule 6 (outside the invention) |
|---|---|---|
| Δa* | -1.1 | |
| b* | 11.7 ± 0.2 | 16.4 ± 0.4 |
| **Δb*** | **4.7** | |
| **ΔE*** | **4.8** | |

The results on the examples 5 and 6 show that the formulation 5 with the compound (2) is significantly less yellow than the equivalent formulation 6 with the compound MC11 of the application WO2008/ 090066.

## Claims

1. Cosmetic or dermatological composition in emulsion form, comprising, in a physiologically acceptable support:

   a) at least one aqueous phase
   b) at least one oily phase
   c) at least one merocyanine compound of formula (1) or one of the E/E- or E/Z-geometrical isomer forms thereof:

   (1) 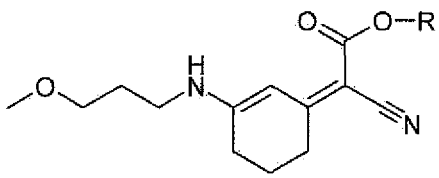

   in which:
   R is a $C_1$-$C_{22}$ alkyl group, a $C_2$-$C_{22}$ alkenyl group, a $C_2$-$C_{22}$ alkynyl group, a $C_3$-$C_{22}$ cycloalkyl group or a $C_3$-$C_{22}$ cycloalkenyl group, the said groups possibly being interrupted with one or more O, and
   d) at least one emulsifying system containing at least one gemini surfactant.

2. Composition according to Claim 1, in which the merocyanine compound(s) are chosen from those in which: R is a $C_1$-$C_{22}$ alkyl, which may be interrupted with one or more O.

3. Composition according to Claim 1 or 2, in which the merocyanine compound(s) are chosen from the following compounds, and also the E/E- or E/Z- geometrical isomer forms thereof:

| | | | |
|---|---|---|---|
| 1 | 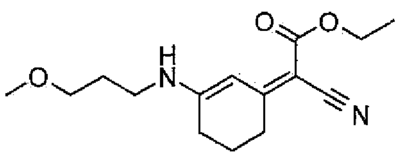 ethyl (2Z)-cyano{3-[(3-methoxypropyl)amino] cyclohex-2-en-1-ylidene}ethanoate | 4 | 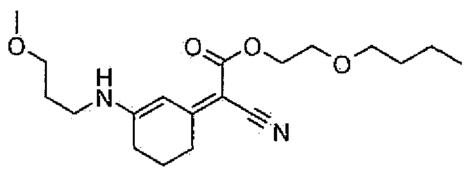 2-butoxyethyl (2Z)-cyano{3-[(3-methoxypropyl) amino]cyclohex-2-en-1-ylidene}ethanoate |
| 2 | 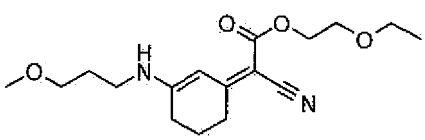 2-ethoxyethyl (2Z)-cyano{3-[(3-methoxypropyl) amino]cyclohex-2-en-1-ylidene}ethanoate | 5 | 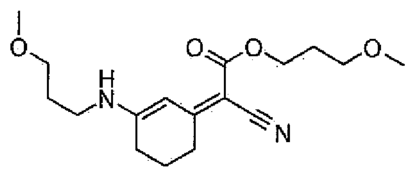 3-methoxypropyl (2Z)-cyano{3-[(3-methoxypropyl) amino]cyclohex-2-en-1-ylidene}ethanoate |

(continued)

| | | | |
|---|---|---|---|
| 3 | 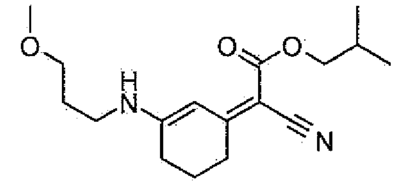 2-methylpropyl (2Z)-cyano{3-[(3-methoxypropyl) amino]cyclohex-2-en-1-ylidene}ethanoate | 6 | 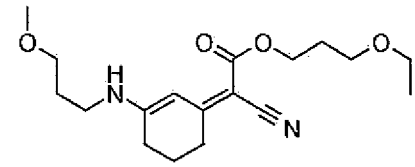 3-ethoxypropyl (2Z)-cyano{3-[(3-methoxypropyl) amino]cyclohex-2-en-1- ylidene}ethanoate |

**4.** Composition according to Claim 3, in which the merocyanine compound is 2-ethoxyethyl (2Z)-cyano{3-[(3-methoxypropyl)amino]cyclohex-2-en-1-ylidene}ethanoate (2) in its E/Z geometrical configuration having the following structure:

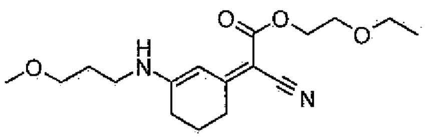

and/or in its E/E geometrical configuration having the following structure:

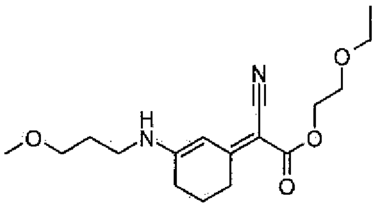

**5.** Composition according to any one of Claims 1 to 4, in which the merocyanine(s) of formula (1) are present in a concentration ranging from 0.1% to 10% by weight and preferentially from 0.2% to 5% by weight relative to the total weight of the composition.

**6.** Composition according to any one of Claims 1 to 5, in which the gemini surfactant(s) correspond to the following general formula:

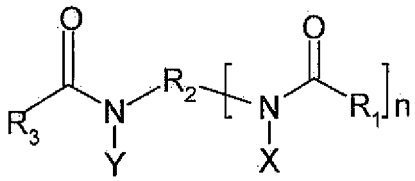

(I)

in which:

$R_1$ and $R_3$ denote, independently of each other, an alkyl radical containing from 1 to 25 carbon atoms;
$R_2$ denotes a spacer consisting of a linear or branched alkylene chain containing from 1 to 12 carbon atoms;
X denotes a group $-(C_2H_4O)_a-(C_3H_6O)_bZ$;
Y denotes a group $-(C_2H_4O)_c-(C_3H_6O)_dZ$;

in which

Z denotes a hydrogen atom or a radical $-CH_2-COOM$, $-SO_3M$, $-P(O)(OM)_2$, $-C_2H_4SO_3M$, $-C_3H_6SO_3M$ or-$CH_2(CHOH)_4CH_2OH$ group, in which M & M' represent H or an alkali metal or alkaline-earth metal or ammonium or alkanolammonium ion,

a and c, independently of each other, range from 0 to 15,
b and d, independently of each other, range from 0 to 10, and
the sum of a + b + c + d ranges from 1 to 25; and
n ranges from 1 to 10.

7. Composition according to Claim 6, in which, in formula (I), $R_1$ and $R_3$ preferably denote, independently of each other, an alkyl radical containing from 5 to 21 and more particularly from 7 to 19 carbon atoms.

8. Composition according to Claim 6 or 7, in which the gemini surfactant is such that each of the groups $R_1$-CO- and $R_3$-CO- comprises from 8 to 20 carbon atoms and preferably denotes a coconut fatty acid residue predominantly comprising lauric acid and myristic acid.

9. Composition according to any one of Claims 6 to 8, in which, in formula (I), b and d are equal to 0 and preferably the sum of a, b, c and d has a mean value ranging from 10 to 20 and is preferably from 12 to 18 and more particularly equal to 15;
preferably, Z is the group $-SO_3M$ in which M is preferably an alkali metal ion such as a sodium ion;
preferably, the spacer $R_2$ consists of a linear $C_1$-$C_3$ alkylene chain and preferably an ethylene chain ($-CH_2CH_2-$);
n is preferentially equal to 0.

10. Composition according to any one of Claims 1 to 9, in which the gemini surfactant is disodium ethylene dicocamide PEG-15 disulfate, having the following structure:

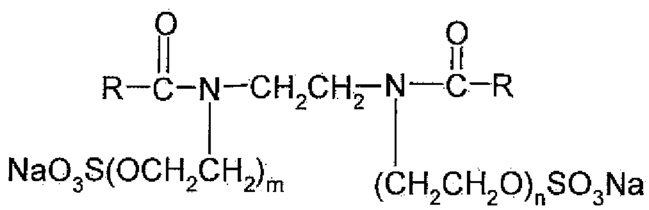

RCO represents a coconut fatty acid radical and m + n has a mean value of 15.

11. Composition according to any one of Claims 1 to 10, in which the gemini surfactant is used as a mixture with other surfactants, and especially as a mixture with (a) a glyceryl ester of a $C_6$-$C_{22}$ fatty acid (preferably $C_{14}$-$C_{20}$ such as a stearate), (b) a diester of a $C_6$-$C_{22}$ fatty acid (preferably $C_{14}$-$C_{20}$ such as a stearate) and of citric acid and of glycerol (especially a diester of a $C_6$-$C_{22}$ fatty acid and of glyceryl monocitrate), and (c) a $C_{10}$-$C_{30}$ fatty alcohol (preferably behenyl alcohol).

12. Composition according to claim 11, in which the gemini surfactant represents from 10% to 20% by weight and advantageously 15% by weight; the glyceryl ester of a $C_6$-$C_{22}$ fatty acid represents from 30% to 40% by weight, advantageously 35% by weight; the diester of a $C_6$-$C_{22}$ fatty acid and of citric acid and of glycerol represents from 10% to 20% by weight, advantageously 15% by weight; and the $C_{10}$-$C_{30}$ fatty alcohol represents from 30% to 40% by weight, advantageously 35% by weight, relative to the total weight of the mixture of surfactants comprising the gemini surfactant.

13. Composition according to Claim 11 or 12, comprising a mixture of disodium ethylene dicocamide PEG-15 disulfate, glyceryl stearate, glyceryl stearate monocitrate, behenyl alcohol, and preferably a mixture of from 10% to 20% by weight of disodium ethylene dicocamide PEG-15 sulfate, from 30% to 40% by weight of glyceryl stearate, from 10% to 20% by weight of glyceryl stearate monocitrate, and from 30% to 40% by weight of behenyl alcohol, relative to the total weight of the mixture of surfactants comprising the gemini surfactant.

14. Composition according to any one of Claims 1 to 10, in which the gemini surfactant is used as a mixture with an anionic surfactant such as a lauric acid ester, sodium lauroyl lactate.

15. Composition according to any one of the preceding claims, comprising the following mixtures of surfactants in which the gemini surfactant represents from 3% to 50% of the weight of these mixtures:

behenyl alcohol, glyceryl stearate, glyceryl stearate citrate and disodium ethylene dicocamide PEG-15 disulfate,

sodium lauroyl lactylate and disodium ethylene dicocamide PEG-15 disulfate capric/caprylic triglyceride, ceteareth-25, disodium ethylene dicocamide PEG-15 disulfate, sodium lauroyl lactylate, behenyl alcohol, glyceryl stearate, glyceryl stearate citrate.

**16.** Composition according to any one of the preceding claims, in which the gemini surfactant(s) are present in an active material content ranging from 0.05% to 10% by weight, preferably ranging from 0.1% to 5% by weight and better still ranging from 0.2% to 2% by weight, relative to the total weight of the composition.

**17.** Composition according to any one of the preceding claims, in which the composition is in the form of an oil-in-water emulsion.

**18.** Composition as defined in any of the preceding claims for use in a method for caring for and/or making up a keratin material, comprising the application, to the surface of the said keratin material of the said composition.

**19.** Composition as defined in any of the preceding claims for use in a method for limiting the darkening of the skin and/or improving the colour and/or uniformity of the complexion, comprising the application, to the surface of the skin of the said composition.

**20.** Composition as defined in any of the preceding claims for use in a method for preventing and/or treating the signs of ageing of a keratin material, comprising the application, to the surface of the keratin material of the said composition.

**Patentansprüche**

**1.** Kosmetische oder dermatologische Zusammensetzung in Emulsionsform, umfassend in einem physiologisch annehmbaren Träger:

a) mindestens eine wässrige Phase,
b) mindestens eine ölige Phase,
c) mindestens eine Merocyaninverbindung mit der Formel (1) oder eine der geometrischen E/E- oder E/Z-Isomerformen davon:

(1) 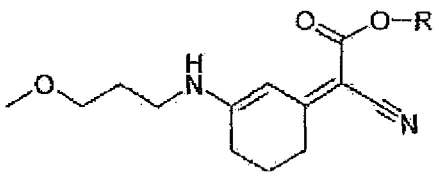

wobei:
R eine $C_1$-$C_{22}$-Alkylgruppe, eine $C_2$-$C_{22}$-Alkenylgruppe, eine $C_2$-$C_{22}$-Alkinylgruppe, eine $C_3$-$C_{22}$-Cycloalkylgruppe oder eine $C_3$-$C_{22}$-Cycloalkenylgruppe ist, wobei diese Gruppen möglicherweise durch ein oder mehrere O unterbrochen sein können, und
d) mindestens ein Emulgiersystem, das mindestens ein Gemini-Tensid enthält.

**2.** Zusammensetzung nach Anspruch 1, wobei die Merocyaninverbindung (en) ausgewählt ist bzw. sind aus jenen, worin:
R ein $C_1$-$C_{22}$-Alkyl ist, das durch ein oder mehrere O unterbrochen sein kann.

**3.** Zusammensetzung nach Anspruch 1 oder 2, wobei die Merocyaninverbindung(en) ausgewählt ist bzw. sind aus den folgenden Verbindungen und auch den geometrischen E/E- oder E/Z-Isomerformen davon:

| | | | |
|---|---|---|---|
| 1 | 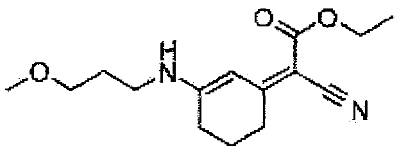<br>Ethyl(2Z)-cyano{3-[(3-methoxypropyl)amino] cyclohex-2-en-1-yliden}ethanoat | 4 | 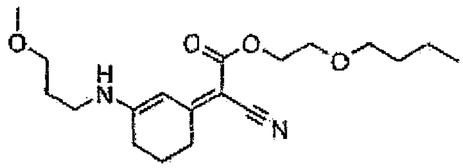<br>2-Butoxyethyl(2Z)-cyano{3- [(3-methoxypropyl) amino]cyclohex-2-en-1-yliden}ethanoat |
| 2 | 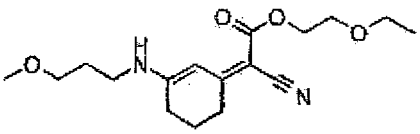<br>2-Ethoxyethyl(2Z)-cyano(3-[(3-methoxypropyl) amino]cyclohex-2-en-1-yliden}ethanoat | 5 | 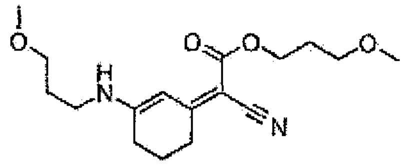<br>3-Methoxypropyl(2Z)-cyano{3-[(3-methoxypropyl) amino]cyclohex-2-en-1-yliden)ethanoat |
| 3 | 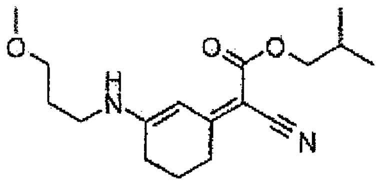<br>2-Methylpropyl(2Z)-cyano{3-[{3-methoxypropyl) amino]cyclohex-2-en-1-yliden}ethanoat | 6 | 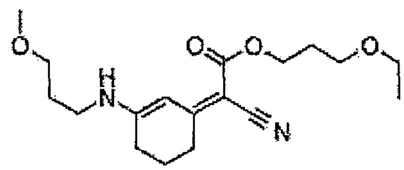<br>3-Ethoxypropyl(2Z)-cyano{3-[(3-methoxypropyl) amino]cyclohex-2-en-1-yliden}ethanoat |

4. Zusammensetzung nach Anspruch 3, wobei die Merocyaninverbindung 2-Ethoxyethyl-(2z)-cyano{3-[{3-methoxypropyl)amino]cyclohex-2-en-1-yliden}ethanoat (2) in seiner geometrischen E/Z-Konfiguration mit der folgenden Struktur ist:

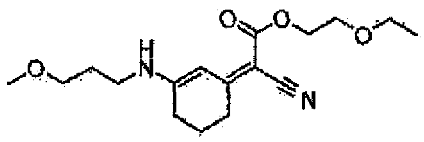

und/oder in seiner geometrischen E/E-Konfiguration mit der folgenden Struktur:

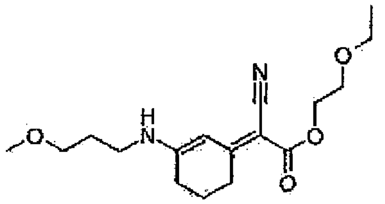

5. Zusammensetzung nach einem der Ansprüche 1 bis 4, wobei das Merocyanin/die Merocyanine der Formel (1) in einer Konzentration im Bereich von 0,1 Gew.% bis 10 Gew.% und vorzugsweise 0,2 Gew.% bis 5 Gew.% vorhanden ist bzw. sind, bezogen auf das Gesamtgewicht der Zusammensetzung.

6. Zusammensetzung nach einem der Ansprüche 1 bis 5, wobei das Gemini-Tensid/die Gemini-Tenside der folgenden allgemeinen Formel entspricht bzw. entsprechen:

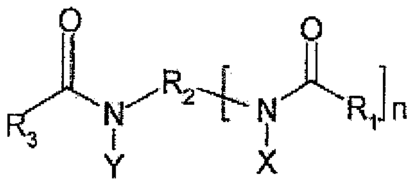

(I)

wobei:

$R_1$ und $R_3$ unabhängig voneinander einen Alkylrest bezeichnen, der 1 bis 25 Köhlenstoffatome enthält;
$R_2$ einen Spacer bezeichnet, der aus einer linearen oder verzweigten Alkylenkette besteht, die 1 bis 12 Kohlenstoffatome enthält;
X eine Gruppe $-(C_2H_4O)_a-(C_3H_6O)_bZ$ bezeichnet;
Y eine Gruppe $-(C_2H_4O)_c-(C_3H_6O)_dZ$ bezeichnet; worin
Z ein Wasserstoffatom oder einen Rest $-CH_2-COOM$, $-SO_3M$, $-P(O)(OM)_2$, $-C_2H_4SO_3M$, $-C_3H_6SO_3M$ oder eine Gruppe $-CH_2(CHOH)_4CH_2OH$ bezeichnet, wobei M und M' für H oder ein Alkalimetall- oder Erdalkalimetall- oder Ammonium- oder Alkanolammoniumion stehen,
a und c unabhängig voneinander im Bereich von 0 bis 15 liegen,
b und d unabhängig voneinander im Bereich von 0 bis 10 liegen, und
die Summe aus a + b + c + d im Bereich von 1 bis 25 liegt; und
n im Bereich von 1 bis 10 liegt.

7. Zusammensetzung nach Anspruch 6, wobei $R_1$ und $R_3$ in Formel (I) vorzugsweise unabhängig voneinander einen Alkylrest bezeichnen, der 5 bis 21 und insbesondere 7 bis 19 Kohlenstoffatome enthält.

8. Zusammensetzung nach Anspruch 6 oder 7, wobei das Gemini-Tensid derart ist, dass jede der Gruppen $R_1$-CO- und $R_3$-Co- 8 bis 20 Kohlenstoffatome umfasst und vorzugsweise einen Kokosfettsäurerest bezeichnet, der vorwiegend Laurinsäure und Myristinsäure umfasst.

9. Zusammensetzung nach einem der Ansprüche 6 bis 8, wobei in Formel (I) b und d gleich 0 sind und vorzugsweise die Summe aus a, b, c und d einen Mittelwert im Bereich von 10 bis 20 aufweist und vorzugsweise 12 bis 18 und insbesondere gleich 15 ist;
vorzugsweise Z die Gruppe $-SO_3M$ ist, in der M vorzugsweise ein Alkalimetallion ist, wie ein Natriumion;
der Spacer $R_2$ vorzugsweise aus einer linearen $C_1$-$C_3$-Alkylenkette und vorzugsweise einer Ethylenkette ($-CH_2CH_2-$) besteht;
n vorzugsweise gleich 0 ist.

10. Zusammensetzung nach einem der Ansprüche 1 bis 9, wobei das Gemini-Tensid Dinatrium-Ethylendicocamid-PEG-15-disulfat mit der folgenden Struktur ist:

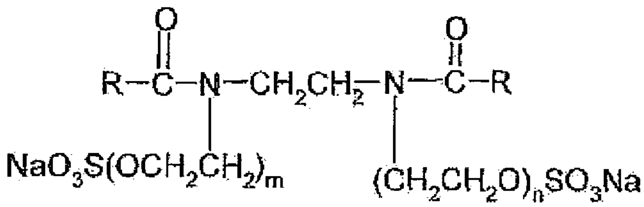

RCO für einen Kokosfettsäurerest steht und m + n einen Mittelwert von 15 hat.

11. Zusammensetzung nach einem der Ansprüche 1 bis 10, wobei das Gemini-Tensid als Mischung mit anderen Tensiden verwendet wird, und insbesondere als Mischung mit (a) einem Glycerylester einer $C_6$-$C_{22}$-Fettsäure (vorzugsweise $C_{14}$-$C_{20}$, wie einem Stearat), (b) einem Diester einer $C_6$-$C_{22}$-Fettsäure (vorzugsweise $C_{14}$-$C_{20}$, wie einem Stearat) und von Citronensäure und Glycerin (insbesondere einem Diester einer $C_6$-$C_{22}$-Fettsäure und von Glycerylmonocitrat), und (c) einem $C_{10}$-$C_{30}$-Fettalkohol (vorzugsweise Behenylalkohol).

12. Zusammensetzung nach Anspruch 11, wobei das Gemini-Tensid 10 Gew.% bis 20 Gew.% und vorteilhaft 15 Gew.% stellt; der Glycerylester einer $C_6$-$C_{22}$-Fettsäure 30 Gew.% bis 40 Gew.% stellt, vorzugsweise 35 Gew.%; der Diester

einer $C_6$-$C_{22}$-Fettsäure und von Citronensäure und Glycerin 10 Gew.% bis 20 Gew.% stellt, vorteilhaft 15 Gew.%; und der $C_{10}$-$C_{30}$-Fettalkohol 30 Gew.% bis 40 Gew.% stellt, vorteilhaft 35 Gew.%, bezogen auf das Gesamtgewicht der Mischung von Tensiden, die das Gemini-Tensid umfasst.

**13.** Zusammensetzung nach Anspruch 11 oder 12, umfassend eine Mischung von Dinatrium-Ethylendicocamid-PEG-15-disulfat, Glycerylstearat, Glycerylstearatmonocitrat, Behenylalkohol, und vorzugsweise eine Mischung aus 10 Gew.% bis 20 Gew.% Dinatrium-Ethylendicocamid-PEG-15-sulfat, 30 Gew.%bis 40 Gew.% Glycerylstearat, 10 Gew.% bis 20 Gew.% Glycerylstearatmonocitrat und 30 Gew.% bis 40 Gew.% Behenylalkohol, bezogen auf das Gesamtgewicht der Mischung von Tensiden, die das Gemini-Tensid umfasst.

**14.** Zusammensetzung nach einem der Ansprüche 1 bis 10, wobei das Gemini-Tensid als Mischung mit einem anionischen Tensid verwendet wird, wie einem Laurinsäureester, Natriumlauroyllactat.

**15.** Zusammensetzung nach einem der vorhergehenden Ansprüche, umfassend die folgenden Mischungen von Tensiden, wobei das Gemini-Tensid 3 % bis 50 % des Gewichts dieser Mischungen stellt:

Behenylalkohol, Glycerylstearat, Glycerylstearatcitrat und Dinatriuin-Ethylendicocamid-PEG-15-disulfat,
Natriumlauroyllactylat und Dinatrium-Ethylendicocamid-PEG-15-disulfat,
Caprin-/Capryltriglycerid, Ceteareth-25, Dinatrium-Ethylendicocamid-PEG-15-disulfat, Natriumlauroyllactylat, Behenylalkohol, Glycerylstearat, Glycerylstearatcitrat.

**16.** Verfahren nach einem der vorhergehenden Ansprüche, wobei das Gemini-Tensid/die Gemini-Tenside in einem aktiven Materialgehalt im Bereich von 0,05 Gew.% bis 10 Gew.%, vorzugsweise im Bereich von 0,1 Gew.% bis 5 Gew. % und noch besser im Bereich von 0,2 Gew.% bis 2 Gew.% vorhanden ist bzw. sind, bezogen auf das Gesamtgewicht der Zusammensetzung,

**17.** Zusammensetzung nach einem der vorhergehenden Ansprüche, wobei die Zusammensetzung in Form einer Öl-in-Wasser-Emulsion vorliegt.

**18.** Zusammensetzung nach einem der vorhergehenden Ansprüche zur Verwendung in einem Verfahren zum Pflegen und/oder Herrichten eines Keratinmaterials, umfassend das Auftragen der Zusammensetzung auf die Oberfläche des Keratinmaterials.

**19.** Zusammensetzung nach einem der vorhergehenden Ansprüche zur Verwendung in einem Verfahren zur Begrenzung des Dunkelwerdens der Haut und/oder zum Verbessern der Farbe und/oder der Gleichförmigkeit der Hautfarbe, umfassend Auftragen der Zusammensetzung auf die Oberfläche der Haut.

**20.** Zusammensetzung nach einem der vorhergehenden Ansprüche zur Verwendung in einem Verfahren zum Verhindern und/oder Behandeln der Anzeichen von Alterung eines Keratinmaterials, umfassend das Auftragen der Zusammensetzung auf die Oberfläche des Keratinmaterials.

## Revendications

**1.** Composition cosmétique ou dermatologique sous forme d'émulsion comprenant dans un support physiologiquement acceptable :

a) au moins une phase aqueuse
b) au moins une phase huileuse
c) au moins un composé mérocyanine de formule (1) ou l'une de ses formes géométriques isomères E/E- ou E/Z-:

(1)

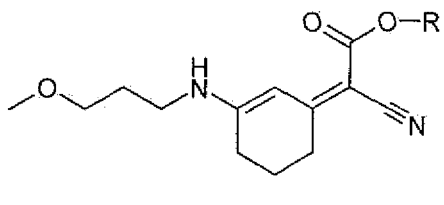

dans laquelle

R est un groupement alkyle en $C_1$-$C_{22}$, un groupement alcényle en $C_2$-$C_{22}$, un groupement alcinyle en $C_2$-$C_{22}$, un groupement cycloalkyle en $C_3$-$C_{22}$ ou un groupement cycloalcényle en $C_3$-$C_{22}$, lesdits groupements pouvant être interrompus par un ou plusieurs O et

d) au moins un système émulsionnant contenant au moins un tensioactif géminé.

**2.** Composition selon la revendication 1, où le ou les composés mérocyanines sont choisis parmi ceux où R est un alkyle en $C_1$-$C_{22}$, pouvant être interrompu par un ou plusieurs O.

**3.** Composition selon la revendication 1 ou 2, où le ou les composés mérocyanines sont choisis parmi les composés suivants ainsi que leurs formes géométriques isomères E/E- ou E/Z- :

| | | | |
|---|---|---|---|
| 1 | 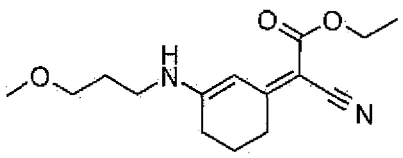 ethyl (2Z)-cyano{3-[(3-methoxypropyl)amino] cyclohex -2-en-1-ylidene}ethanoate | 4 | 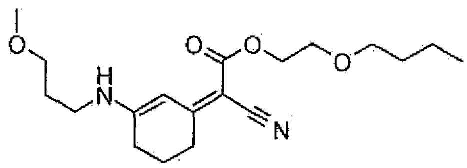 2-butoxyethyl (2Z)-cyano{3-[(3-methoxypropyl) amino]cyclohex -2-en-1-ylidenelethanoate |
| 2 | 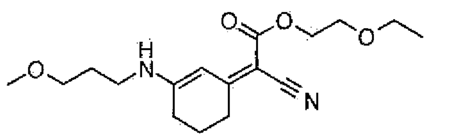 2-ethoxyethyl (2Z)-cyano(3-[(3-methoxypropyl) amino]cyclohex -2-en-1-ylidene}ethanoate | 5 | 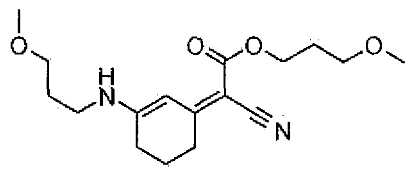 3-methoxypropyl (2Z)-cyano{3-[(3-methoxypropyl) amino]cyclohex -2-en-1-ylidenelethanoate |
| 3 | 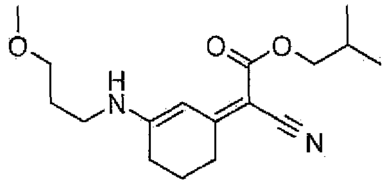 2-methylpropyl(2Z)-cyano{3-[(3-methoxypropyl) amino]cyclohex -2-en-1-ylidene}ethanoate | 6 | 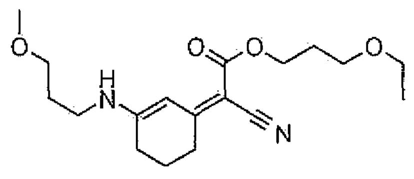 3-ethoxypropyl (2Z)-cyano{3-[{3-methoxypropyl) amino]cyclohex -2-en-1-ylidenelethanoate |

**4.** Composition selon la revendication 3, où lé composé mérocyanine est le 2-éthoxyéthyle (22)-cyano{3-[(3-méthoxypropyl)-amino]cyclohex-2-èn-1-ylidène}éthanoate (2) dans sa configuration géométrique E/Z de structure suivante :

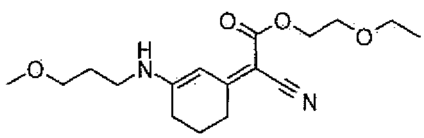

et/ou dans sa configuration géométrique E/E de structure suivante :

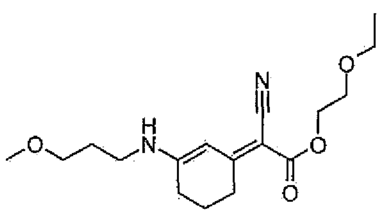

5. Composition selon l'une quelconque des revendications 1 à 4, où le ou les mérocyanines de formule (1) sont présentes dans une concentration allant de 0,1% à 10% en poids et préférentiellement de 0,2% à 5% en poids par rapport au poids total de la composition.

6. Composition selon l'une quelconque des revendications 1 à 5, où le ou les tensioactifs géminés répondent à la formule générale suivante ;

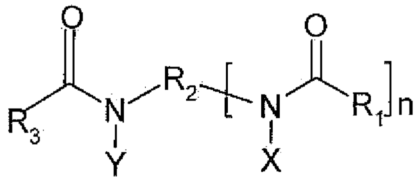

(I)

dans laquelle :

- $R_1$ et $R_3$ désignent, indépendamment l'un de l'autre, un radical alkyle ayant de 1 à 25 atomes de carbone ;
- $R_2$ désigne un espaceur constitué d'une chaîne alkylène linéaire ou ramifiée ayant de 1 à 12 atomes de carbone ;
- X désigne un groupement $-(C_2H_4O)_a-(C_3H_6O)_bZ$
- Y désigne un groupement $-(C_2H_4O)_c-(C_3H_6O)_dZ$

où

Z désigne un atome d'hydrogène ou un radical $-CH_2-COOM$, $-SO_3M$, $-P(O)(OM)_2$,, $-C_2H_4-SO_3M$, $-C_3H_6-SO_3M$ ou $-CH_2(CHOH)_4CH_2OH$, où M et M' représentent H ou un ion alcalin ou alcalino-terreux ou ammonium ou alcanolammonium,
a et c, indépendamment l'un de l'autre, vont de 0 à 15,
b et d, indépendamment l'un de l'autre, vont de 0 à 10, et
la somme de a + b +c + d va de 1 à 25 ; et
n va de 1 à 10.

7. Composition selon la revendication 6, où dans la formule (I), $R_1$ et $R_3$ désignent, indépendamment l'un de l'autre, de préférence un radical alkyle ayant de 5 à 21, et plus particulièrement de 7 à 19 atomes de carbone.

8. Composition selon la revendication 6 ou 7, où le tensioactif géminé est tel que chacun des groupements $R_1$-CO- et $R_3$-CO- comprend de 8 à 20 atomes de carbone, et désigne de préférence un reste d'acides gras de coco comprenant majoritairement de l'acide laurique et de l'acide myristique.

9. Composition selon l'une quelconque des revendications 6 à 8, où dans la formule (I), b et d sont égaux à 0 et de préférence la somme de a, b, c et d a une valeur moyenne allant de 10 à 20 et est de préférence de 12 à 18 et plus particulièrement égale à 15 ;
de préférence Z est le groupement $-SO_3M$ où M est de préférence un ion alcalin tel qu'un ion sodium ;
de préférence l'espaceur $R_2$ est constitué d'une chaîne alkylène linéaire en $C_1$-$C_3$, et de préférence d'une chaîne éthylène ($-CH_2CH_2-$) ;
n est préférentielement égal à 1.

10. Composition selon l'une quelconque des revendications 1 à 9, où le tensioactif géminé est le disodium ethylene dicocamide peg-15 disulfate, ayant la structure suivante :

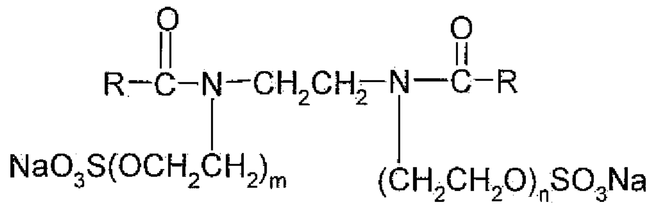

RCO représente un radical d'acides gras de coco et m+n a une valeur moyenne de 15.

**11.** Composition selon l'une quelconque des revendications 1 à 10, où le tensioactif géminé est utilisé en mélange avec d'autres tensioactifs, et notamment en mélange avec (a) un ester d'acide gras en $C_6$-$C_{22}$ (de préférence en $C_{14}$-$C_{20}$ tel qu'un stéarate) et de glycéryle, (b) un diester d'acide gras en $C_6$-$C_{22}$ (de préférence en $C_{14}$-$C_{20}$ tel qu'un stéarate) et d'acide citrique et de glycérol (notamment un diester d'acide gras en $C_6$-$C_{22}$ et de monocitrate de glycéryle), et (c) un alcool gras en $C_{10}$-$C_{30}$ (de préférence l'alcool béhénylique).

**12.** Composition selon la revendication 11, où le tensioactif géminé représente de 10 à 20 % en poids, et avantageusement 15 % en poids ; l'ester d'acide gras en $C_6$-$C_{22}$ et de glycéryle représente de 30 à 40 % en poids, avantageusement 35 % en poids ; le diester d'acide gras en $C_6$-$C_{22}$ et d'acide citrique et de glycérol représente de 10 à 20 % en poids, avantageusement 15 % en poids ; et l'alcool gras en $C_{10}$-$C_{30}$ représente de 30 à 40 % en poids, avantageusement 35 % en poids, par rapport au poids total du mélange de tensioactifs comprenant le tensioactif géminé.

**13.** Composition selon la revendication 11 ou 12, comprenant un mélange de disodium ethylene dicocamide PEG-15 disulfate, de stéarate de glycéryle, de stéarate de monocitrate de glycéryle, d'alcool béhénylique et de préférence un mélange de 10 à 20 % en poids de disodium ethylene dicocamide PEG-15 disulfate, de 30 à 40 % en poids de stéarate de glycéryle, de 10 à 20 % en poids de stéarate de monocitrate de glycéryle, de 30 à 40 % en poids d'alcool béhénylique, par rapport au poids total du mélange de tensioactifs contenant le tensioactif géminé.

**14.** Composition selon l'une quelconque des revendications 1 à 10, où le tensioactif géminé est utilisé en mélange avec un tensioactif anionique tel qu'un ester d'acide laurique, le lauroyl lactate de sodium.

**15.** Composition selon l'une quelconque des revendications précédentes, comprenant les mélanges de tensioactifs suivants dans lesquels le tensioactif géminé représente de 3 à 50% du poids de ces mélanges :

behenyl alcohol, glyceryl stearate, glyceryl stéarate citrate et disodium ethylene dicocamide PEG-15 disulfate,
sodium lauroyl lactylate et disodium ethylene dicocamide PEG-15 disulfate.
capric/caprylic triglycéride, ceteareth-25, disodium ethylene dicocamide PEG-15 disulfate, sodium lauroyl lactylate, behenyl alcohol, glyceryl stearate, glyceryl stearate citrate.

**16.** Composition selon l'une quelconque des revendications précédentes, où le ou les tensioactifs géminés sont présents en une teneur en matière active allant de 0,05 à 10 % en poids, par rapport au poids total de la composition, de préférence allant de 0,1 à 5 % en poids et mieux allant de 0,2 à 2% en poids.

**17.** Composition selon l'une quelconque des revendications précédentes, où là composition se présente sous la forme d'une émulsion huile-dans-eau.

**18.** Composition telle que définie dans l'une quelconque des revendications précédentes pour utilisation dans un procédé de soin et/ou de maquillage d'une matière kératinique comprenant l'application sur la surface de ladite matière kératinique de ladite composition.

**19.** Composition telle que définie dans l'une quelconque des revendications précédentes pour utilisation dans un procédé pour limiter l'assombrissement de la peau et/ou améliorer la couleur et/ou l'homogénéité du teint comprenant l'application sur la surface de la peau de ladite composition.

**20.** Composition telle que définie dans l'une quelconque des revendications précédentes pour utilisation dans un procédé pour prévenir et/ou traiter les signes du vieillissement d'une matière kératinique l'application sur la surface de la matière kératinique de ladite composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4195999 A
- WO 2004006878 A
- WO 2008090066 A
- WO 2011113718 A
- WO 2009027258 A
- WO 2013010590 A
- WO 2013011094 A
- WO 2013011480 A
- WO 2007071582 A
- US 4749643 A
- DE 19943681
- WO 9614926 A
- WO 2007068371 A
- WO 2008155059 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 2007071584 A
- WO 2009063392 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0841341 A
- EP 0518773 A
- FR 2315991
- FR 2416008
- US 4077441 A
- US 4850517 A


### Non-patent literature cited in the description


- *IP COM JOURNAL,* 23 February 2009
- *IP COM JOURNAL*
- *IP COM JOURNAL,* 12 November 2009
- *IP COM Journal,* 03 April 2004
- **W.C. GRIFFIN.** Classification of Surface Active Agents by HLB. *Journal of the Society of Cosmetic Chemists,* 1949, vol. 1, 311
- Calculation of HLB of Non Ionic Surfactants. *Journal of the Society of Cosmetic Chemists,* 1954, vol. 5, 249
- **MILTON J. ROSEN.** Gemini Surfactants, Properties of surfactant molecules with two hydrophilic groups and two hydrophobic groups. *Cosmetics & Toiletries Magazine,* December 1998, vol. 113, 49-55
- **MILTON J. ROSEN.** Recent Developments in Gemini Surfactants. *Allured's Cosmetics & Toiletries Magazine,* July 2001, vol. 116 (7), 67-70
- *CHEMICAL ABSTRACTS,* 919803-06-8
- Symmetrical Triazine Derivatives. *IP.COM IPCOM000031257 Journal,* 20 September 2004
- **BANGHAM.** Standish and Watkins. *J. Mol. Biol.,* 1965, vol. 13, 238